# EUROPEAN PATENT SPECIFICATION

(11) **EP 4 114 369 B1**
(45) Date of publication and mention of the grant of the patent: **26.08.2026**
(21) Application number: 21710231.8
(22) Date of filing: 03.03.2021
(51) Int. Cl.: A61K 38/47, A61K 38/05, A61K 35/28, A61K 31/00, A61K 38/48, A61P 35/00, A61P 37/00, C07K 16/00

(54) **ENZYMES INACTIVATING SERUM IGG FOR USE IN A CONDITIONING REGIMEN FOR THE TRANSPLANT OF HEMATOPOIETIC STEM AND PROGENITOR CELLS**
ENZYME ZUR INAKTIVIERUNG VON SERUM IGG ZUR VERWENDUNG IN EINEM KONDITIONIERUNGSREGIME FÜR DIE TRANSPLANTATION HÄMATOPOETISCHER STAMM UND PROGENITORZELLEN
ENZYMES INACTIVANT L'IGG SÉRIQUE POUR UNE UTILISATION DANS UN RÉGIME DE CONDITIONNEMENT DESTINÉ À LA TRANSPLANTATION DE CELLULES SOUCHES ET PROGÉNITRICES HÉMATOPOÏÉTIQUES

(30) Priority: 04.03.2020 GB 202003129
(43) Date of publication of application: 11.01.2023
(73) Proprietor: Hansa Biopharma AB, 220 07 Lund (SE)
(72) Inventor: BOCKERMANN, Robert, 220 07 Lund (SE); ROBERTSON, Anna-Karin Louise, 220 07 Lund (SE); LIN, Jiaxin, Edmonton Alberta, T6G 2E1 (CA); ANDERSON, Colin Charles, Edmonton Alberta, T6G 2E1 (CA)
(74) Representative: J A Kemp LLP
(86) International application number: PCT/EP2021/055317
(87) International publication number: WO 2021/175914

(56) References cited:
- WO-A1-2016/128558
- WO-A2-2008/071418
- WO-A2-2016/012285
- ID NIHRIO ET AL: "Imlifidase for kidney transplantation in highly sensitised patients with chronic kidney disease Developer/Company Hansa Biopharma AB", HEALTH TECHNOLOGY BRIEFING, 9 July 2019 (2019-07-09), pages 1 - 11, XP055794182, Retrieved from the Internet <URL:http://www.io.nihr.ac.uk/wp-content/uploads/2019/07/11428-Imlifidase-for-Kidney-Transplantation-V1.0-JUL2019-NONCONF.pdf> [retrieved on 20210412]
- LONZE BONNIE E ET AL: "IdeS (Imlifidase): A Novel Agent That Cleaves Human IgG and Permits Successful Kidney Transplantation Across High-strength Donor-specific Antibody", ANNALS OF SURGERY, LIPPINCOTT WILLIAMS & WILKINS, US, vol. 268, no. 3, 31 August 2018 (2018-08-31), pages 488 - 496, XP009527485, ISSN: 1528-1140, DOI: 10.1097/SLA.0000000000002924
- STANLEY C. JORDAN ET AL: "IgG Endopeptidase in Highly Sensitized Patients Undergoing Transplantation", THE NEW ENGLAND JOURNAL OF MEDICINE, vol. 377, no. 5, 3 August 2017 (2017-08-03), US, pages 442 - 453, XP055527161, ISSN: 0028-4793, DOI: 10.1056/NEJMoa1612567

## Description

### Field of the Invention

The present invention relates to a conditioning regimen for the transplant of a cell, tissue or organ, optionally hematopoietic stem / progenitor cells (HSPC), to a subject as defined in the claims. The invention also relates to methods for the induction of hematopoietic chimerism in a subject as defined in the claims. The invention also relates to an enzyme which inactivates serum IgG molecules for use in the prevention or treatment of a disease or condition in a subject, in which hematopoietic chimerism is induced in order to improve the benefit to the subject of a subsequent therapy as defined in the claims. The subsequent therapy may be a cell, tissue or organ transplant or may a gene therapy administered using genetically modified HSPCs.

### Background of the Invention

Tissue/organ transplants may be injured by acute and/or chronic rejection, which may lead to graft failure. Acute and chronic rejection are both typically treated by immunosuppressive agents that can increase the risk of infection, increase the risk of cancer, and also cause organ failure (including of the graft). A technique which can reduce the need for immunosuppression (by establishing immunological tolerance of a transplant) is the induction of hematopoietic chimerism through transplantation from the same donor of hematopoietic stem and progenitor cells, typically in a bone marrow transplant (BMT) before the transplant of cell, tissue or organ. Induced chimerism essentially results in a chimeric immune system which will not attack a graft having the same immunological profile as the donor hematopoietic system, whilst otherwise retaining the recipient's normal immunocompetence to respond to unrelated antigens.

Unfortunately, the complex immunology involved in the transplant of HSPC can be problematic, particularly if there is sensitization to donor antigens prior to transplantation. The presence of donor and recipient immune systems can lead to acute and chronic rejection with both humeral and cellular components. Vigorous host versus graft reactions (HVG) and graft versus host disease (GVHD) are both observed. Often, the transplanted cells fail to successfully engraft in the recipient. Current methods seek to address these problems by pre- and post-transplant immunosuppression. Steps carried out pre-transplant may be referred to as a conditioning regimen and may include treatments that are not solely immunosuppressive. For example, radiation may be used to deplete some or all of the existing bone marrow cells in the recipient, creating space for engraftment of the transplanted cells. However, engraftment is frequently unsuccessful. There is a need for improved conditioning regimens for the transplant of HSPC.

### Summary of the Invention

Conditioning regimens for the transplant of hematopoietic stem and progenitor cells (HSPC) typically include T lymphocyte depletion and/or treatments to reduce donor specific antibodies (DSA) either directly (e.g. by plasmapheresis or the administration of mismatched platelet transfusion that adsorbs DSA) or indirectly by inhibiting antibody production (e.g. using rituximab or bortezomib). However, existing conditioning regimens are frequently ineffective and engraftment is frequently unsuccessful. This may be because high expression of MHC on bone marrow derived cells may increase sensitivity to any remaining functional DSA.

The present inventors have surprisingly shown that a conditioning regimen including enzymatic inactivation of serum IgG in a subject significantly improves engraftment rates (by contrast to the previously used antibody depletion techniques), and hence is more likely to result in hematopoietic chimerism in the subject.

The present invention provides an enzyme which inactivates serum IgG molecules in a subject for use in a conditioning regimen for the transplant of HSPC to a subject, comprising administering the enzyme to the subject. The amount of said enzyme administered is preferably sufficient to inactivate all or substantially all IgG molecules present in the serum of the subject.

The conditioning regimen may additionally comprise one or more of:
(a) administration to the subject of a non-lethal dose of irradiation and/or any other agent which depletes the subject's HSPC
(b) administration of an agent to reduce the numbers and/or down-modulate the activity of lymphocytes in the subject, wherein said lymphocytes include:
   i. T cells; and/or
   ii. B cells (optionally including antibody-producing cells);
(c) administration any other agent or regimen which reduces the activity of the immune system, e.g., inhibitors of complement, inhibitors of cytokines, inhibitors of innate immune cells, inducers of tolerance.

The conditioning regimen preferably includes at least (a), but most preferably includes at least (a) and (b).

The present invention also provides an enzyme which inactivates serum IgG molecules in a subject for use in a method for the induction of hematopoietic chimerism in a subject, the method comprising conducting the conditioning regimen of the invention and subsequently administering HSPC to the subject in an amount sufficient and under conditions suitable to induce hematopoietic chimerism in the subject as defined in the claims. The HSPC may be autologous (the subject's own cells are used) or allogeneic (the cells come from a separate donor). The HSPC may be genetically modified, in which case they are preferably autologous. The genetic modification may be to express any gene, but is typically a gene of therapeutic benefit to the recipient, in which case the HSPC may be referred to as expressing a gene therapy. The HSPC are preferably allogeneic or genetically modified autologous cells. The HSPC are most preferably allogeneic.

The present invention also provides an enzyme which inactivates serum IgG molecules in a subject for use in a method for the prevention or treatment of a disease or condition in a subject, in which hematopoietic chimerism is induced in the subject in accordance with the method of the invention and as defined in the claims in order to improve the benefit to the subject of a therapy for said disease or condition. Said therapy may be a cell, tissue or organ transplant, typically from the same donor as the HSPC. The cell, tissue or organ transplanted may be of any type, including kidney, liver, heart, pancreas, lung, small intestine, skin, blood vessels/vascular tissue, face, arm, trachea, parts of the eye, pancreatic islets, substantia nigra, bone marrow, or stem cells. The cell transplanted may be of any type, including the same HSPC as are used in the method itself, such that no additional therapy is required.

In other words, the invention also provides an enzyme which inactivates serum IgG molecules in a subject for use in a method for the prevention or treatment of immune rejection of a cell, tissue or organ transplant, the method comprising inducing hematopoietic chimerism in the subject in accordance with the method of the invention and as defined in the claims and administering a cell, tissue or organ transplant to the subject, optionally wherein said cell, tissue or organ is from the same donor as the HSPC. The cell, tissue or organ is typically administered after the induction of hematopoietic chimerism in the subject. The cell, tissue or organ transplant may be of any type, including kidney, liver, heart, pancreas, lung, small intestine, skin, blood vessels/vascular tissue, face, arm, trachea, parts of the eye, pancreatic islets, substantia nigra, bone marrow, or stem cells. The cell transplanted may be of any type, including the same HSPC as are used to induce hematopoietic chimerism, such that no additional transplant is required.

Expressed another way, the invention provides a method for the prevention or treatment of immune rejection of a cell, tissue or organ transplant, comprising
(i) conducting the conditioning regimen of the invention;
(ii) administering HSPC to the subject in an amount sufficient and under conditions suitable to induce hematopoietic chimerism in the subject; and
(iii) administering a cell, tissue or organ transplant to the subject from the same donor as the HSPC, optionally wherein said transplant is the administration of HSPC in step (ii).

Where the cell, tissue or organ in step (iii) is the HSPC of step (ii), the enzyme may be used in a method for the treatment of a disease or condition which is treated by HSPC transplant. Where the HSPC are genetically modified to administer a gene therapy, the enzyme may be for the prevention or treatment of the disease or condition to which said gene therapy is directed.

### Brief Description of the Figures

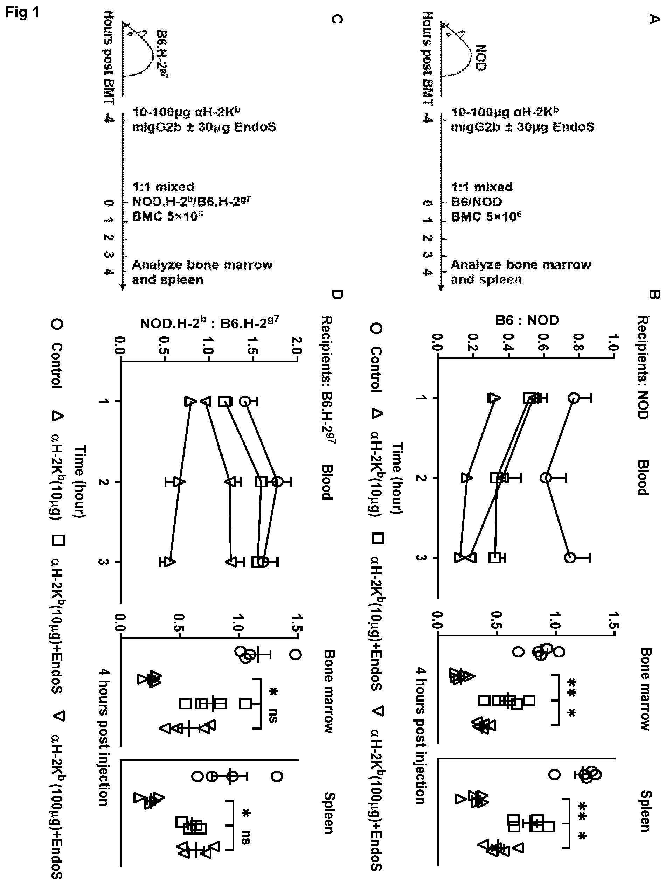

**Fig 1****. EndoS inhibits monoclonal DSA mediated killing of donor bone marrow cells.** Naive NOD (panel A-B) or B6.H-2^{g7} (panel C-D) were given 30µg EndoS and/or anti-H-2K^{b} mAb (10µg or 100µg) intravenously four hours prior to the infusion of a mixture of CFSE labeled NOD/CTV labeled B6 bone marrow cells (BMC; panel A-B) or CFSE labeled B6.H-2^{g7}/CTV labeled NOD.H-2^{b} BMC (panel C-D). Shown are experiment protocols for NOD (panel A) and B6.H-2^{g7} (panel C). Shown are the ratios of dye labeled B6 to NOD cells (panel B) or NOD.H-2^{b} to B6.H-2^{g7} cells (panel D) in the blood (left panels) collected one to three hours after bone marrow transplant (BMT), in host spleens (middle panels) and bone marrow (BM, right panels) collected at four hours after BMT. Mean±SEM are shown. Data were pooled from five (panel B) and four (panel D) independent experiments. Mann-Whitney U test (middle and right panels) was used for the comparisons shown; *p<0.05 and **p<0.01.

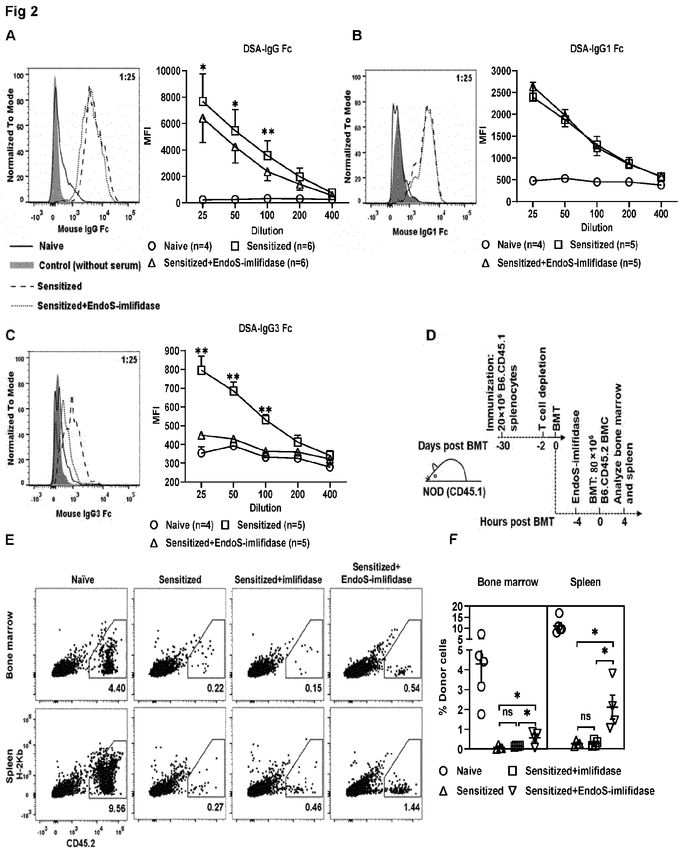

**Fig 2****. EndoS-imlifidase reduces DSA-mediated killing of donor BMC in sensitized recipients.**
   (A-C) Naive NOD mice were immunized with FVB splenocytes four weeks prior to the administration of EndoS-imlifidase. Sera were harvested prior to immunization, prior to and four hours after enzyme treatment. Representative histograms on the left are for DSA-IgG Fc (panel A), DSA-IgG₁ Fc (panel B), DSA-IgG₃ Fc (panel C) and DSA-IgG₃ heavy chain (panel D) with sera at a 1:25 dilution. Mean fluorescence intensity (MFI) of DSA in the titrated sera is shown on the right. Mean±SEM are shown. Ratio paired t test was used to compare MFI of DSA before and after enzyme treatment at each serum dilution with *p<0.05, **p<0.01. (D) Schematic of the experiment shown in E-F. Naive NOD mice were immunized with B6.CD45.1 splenocytes four weeks prior to injection of T cell depleting mAbs. EndoS-imlifidase was administrated two days post T cells depletion. Four hours after enzyme treatment, NOD mice were injected with 80 million B6.CD45.2 bone marrow cells intravenously. Splenocytes and bone marrow cells were analysed for the expression of MHC-I H-2K^{b} and CD45.2. (E-F) Shown are representative dot plots of the four different treatment groups (on the left) and the percentage of donor cells (on the right, mean±SEM). One-way ANOVA with Holm-Sidak's multiple comparisons were used to compare values between the three sensitized groups with *p<0.05.

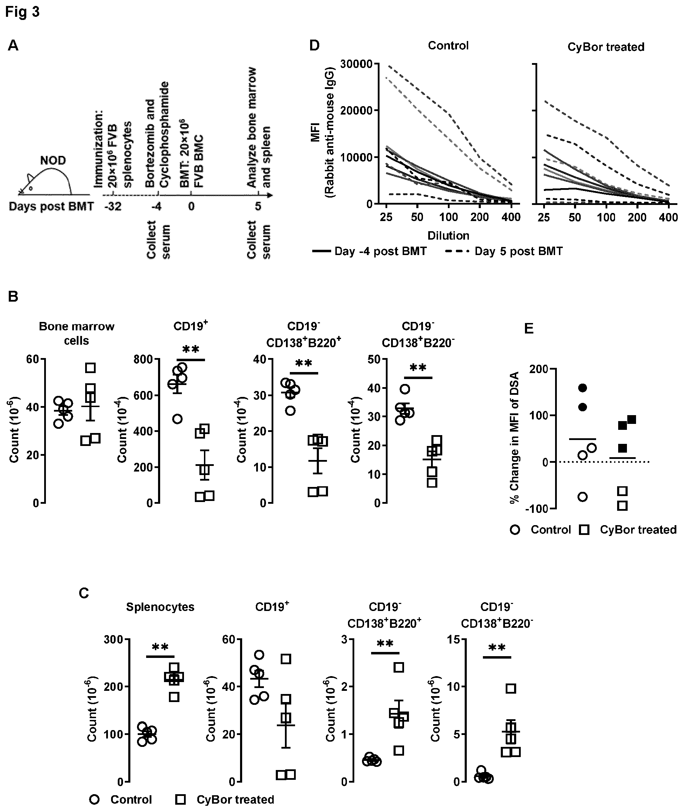

**Fig 3****. Bortezomib/Cyclophosphamide prior to BMT reduces Bone Marrow B cells in sensitized recipients.**
   (A) Schematic of the experiment shown in B-E. Four weeks after immunization with FVB splenocytes, NOD mice were treated with cyclophosphamide and bortezomib (CyBor) intravenously. Four days after CyBor treatment, bone marrow transplantation with 20 million FVB BMC was done. Splenocytes and bone marrow cells were collected five days after BMT for analysis. Sera were collected before CyBor treatment and five days post BMT. Shown are cell counts of B cells and plasma cells in the bone marrow (panel B) and spleens (panel C) in mice given CyBor or vehicle. (D) Sera were collected prior to immunization and five days post BMT, i.e. nine days after CyBor treatment. Shown are MFI of DSA-IgG Fc in the titrated sera from individual control (on the left) or CyBor treated mice (on the right). (E) Shown are percentile changes at day 9 in MFI of DSA at the 1:25 dilution compared to pretreatment. Filled and empty symbols represent data collected in two separate experiments.

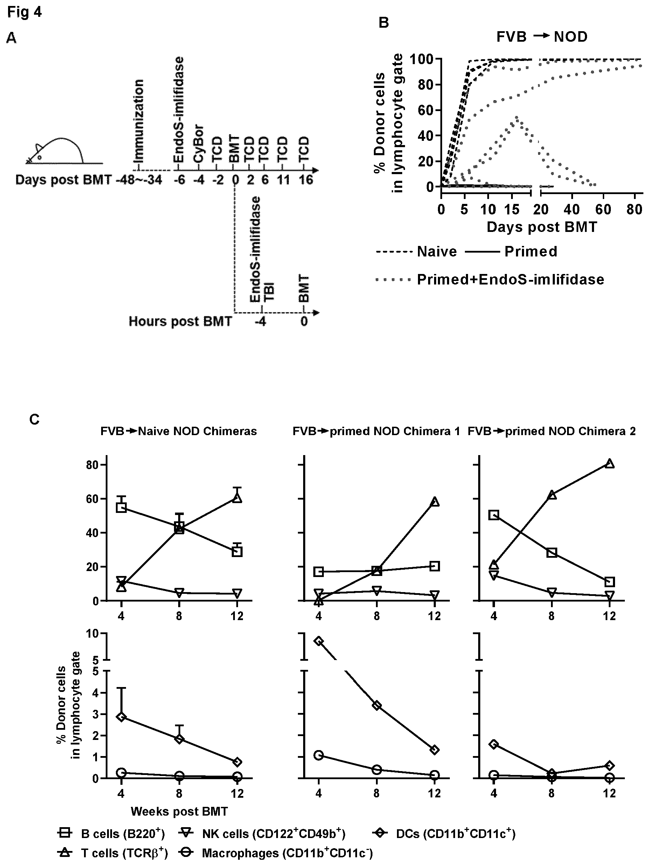

**Fig 4****. EndoS-imlifidase allows hematopoietic chimerism in pre-sensitized recipients**
   (A) Schematic of the chimerism induction protocol; naive B6.H-2^{g7} or NOD mice were immunized with FVB splenocytes four to six weeks prior to chimerism induction. For chimerism induction, CyBor was given on day -4 with respect to the date of BMT. T cell depleting (TCD) antibodies were administered i.p. on day -2, 2, 6, 11, and 16. Some recipients that had been sensitized to FVB splenocytes were treated with EndoS-imlifidase i.v. on day -6 and a repeated dose on day 0 at four hours before BMT. Six Gy total body irradiation was given at 4 hours prior to BMT on day 0. FVB BMC (80×10⁶) were given on day 0. (B) Shown are the proportions of donor cells in lymphocyte gate in peripheral blood over time. (C) Shown are percentages of different lineages of donor cells in lymphocyte gate in peripheral blood from naive NOD chimeras (n=4, on the left, mean±SEM) and primed NOD chimeras (n=2, on the right). Data were pooled from six independent experiments.
**Fig 5****. Mean imlifidase concentration vs. nominal time from dosing** (N=15). Data BLQ are included in mean calculation as BLQ/2. SD indicated with bars.
**Fig 6****. In vitro cleavage of rATG by imlifidase over time.** Columns indicate number of subjects with visible intact rATG on Western blot post-imlifidase (N=11).

### Brief Description of the Sequences

SEQ ID NO: 1 is the full sequence of IdeS including N terminal methionine and signal sequence. It is also available as NCBI Reference sequence no. WP_010922160.1
SEQ ID NO: 2 is the mature sequence of IdeS, lacking the N terminal methionine and signal sequence. It is also available as Genbank accession no. ADF13949.1
SEQ ID NO: 3 is the full sequence of IdeZ including N terminal methionine and signal sequence. It is also available as NCBI Reference sequence no. WP_014622780.1.
SEQ ID NO: 4 is the mature sequence of IdeZ, lacking the N terminal methionine and signal sequence.
SEQ ID NO: 5 is the sequence of a hybrid IdeS/Z. The N terminus is based on IdeZ lacking the N terminal methionine and signal sequence.
SEQ ID NOs: 6 to 25are the sequences of exemplary proteases for use in the methods of the invention.
SEQ ID NO: 26 is the sequence of an IdeS polypeptide. Comprises the sequence of SEQ ID NO: 2 with an additional N terminal methionine and a histidine tag (internal reference pCART124).
SEQ ID NO: 27 is the sequence of an IdeZ polypeptide. Comprises the sequence of SEQ ID NO: 4 with an additional N terminal methionine and a histidine tag (internal reference pCART144).
SEQ ID NO: 28 is the sequence of an IdeS/Z polypeptide. Comprises the sequence of SEQ ID NO: 5 with an additional N terminal methionine and a histidine tag (internal reference pCART145).
SEQ ID NO: 29 is the contiguous sequence PLTPEQFRYNN, which corresponds to positions 63-73 of SEQ ID NO: 3.
SEQ ID NO: 30 is the contiguous sequence PPANFTQG, which corresponds to positions 58-65 of SEQ ID NO: 1.
SEQ ID NO: 31 is the contiguous sequence DDYQRNATEAYAKEVPHQIT, which corresponds to positions 35-54 of SEQ ID NO: 3.
SEQ ID NO: 32 is the contiguous sequence DSFSANQEIRYSEVTPYHVT, which corresponds to positions 30-49 of SEQ ID NO: 1.
SEQ ID NOs: 33 to 55 are nucleotide sequences encoding proteases set out above.
SEQ ID NOs: 56 to 69 are the sequences of exemplary exemplary proteases for use in the methods of the invention.
SEQ ID NO: 70 is the contiguous sequence NQTN, which corresponds to positions 336-339 of SEQ ID NO: 1.
SEQ ID NO: 71 is the contiguous sequence DSFSANQEIR YSEVTPYHVT, which corresponds to positions 30-49 of SEQ ID NO: 1.
SEQ ID NOs: 72 to 86 are nucleotide sequences encoding polypeptides disclosed herein.
SEQ ID NO: 87 is the sequence SFSANQEIRY SEVTPYHVT, which corresponds to positions 31-49 of SEQ ID NO: 1.
SEQ ID NO: 88 is the sequence DYQRNATEAY AKEVPHQIT, which corresponds to positions 36-54 of the IdeZ polypeptide NCBI Reference Sequence no WP_014622780.1.
SEQ ID NO: 89 is the sequence DDYQRNATEA YAKEVPHQIT, which may be present at the N terminus of a polypeptide of the invention.
SEQ ID NO: 90 is the mature sequence of EndoS (Endoglycosidase of S. pyogenes).

### Detailed Description of the Invention

### General

It is to be understood that different applications of the disclosed products and methods may be tailored to the specific needs in the art. It is also to be understood that the terminology used herein is for the purpose of describing particular embodiments of the invention only, and is not intended to be limiting.

In addition as used in this specification and the appended claims, the singular forms "a", "an", and "the" include plural referents unless the content clearly dictates otherwise. Thus, for example, reference to "a polypeptide" includes "polypeptides", and the like.

A "polypeptide" is used herein in its broadest sense to refer to a compound of two or more subunit amino acids, amino acid analogs, or other peptidomimetics. The term "polypeptide" thus includes short peptide sequences and also longer polypeptides and proteins. As used herein, the term "amino acid" refers to either natural and/or unnatural or synthetic amino acids, including both D or L optical isomers, and amino acid analogs and peptidomimetics.

The terms "patient" and "subject" are used interchangeably and typically refer to a human. References to IgG typically refer to human IgG unless otherwise stated.

Amino acid identity as discussed above may be calculated using any suitable algorithm. For example the PILEUP and BLAST algorithms can be used to calculate identity or line up sequences (such as identifying equivalent or corresponding sequences (typically on their default settings), for example as described in Altschul S. F. (1993) J Mol Evol 36:290-300; Altschul, S, F et al (1990) J Mol Biol 215:403-10. Software for performing BLAST analyses is publicly available through the National Center for Biotechnology Information (This algorithm involves first identifying high scoring sequence pair (HSPs) by identifying short words of length W in the query sequence that either match or satisfy some positive-valued threshold score T when aligned with a word of the same length in a database sequence. T is referred to as the neighbourhood word score threshold (Altschul *et al,* supra). These initial neighbourhood word hits act as seeds for initiating searches to find HSPs containing them. The word hits are extended in both directions along each sequence for as far as the cumulative alignment score can be increased. Extensions for the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLAST program uses as defaults a word length (W) of 11, the BLOSUM62 scoring matrix (see Henikoff and Henikoff (1992) Proc. Natl. Acad. Sci. USA 89: 10915-10919) alignments (B) of 50, expectation (E) of 10, M=5, N=4, and a comparison of both strands.

The BLAST algorithm performs a statistical analysis of the similarity between two sequences; see e.g., Karlin and Altschul (1993) Proc. Natl. Acad. Sci. USA 90: 5873-5787. One measure of similarity provided by the BLAST algorithm is the smallest sum probability (P(N)), which provides an indication of the probability by which a match between two polynucleotide or amino acid sequences would occur by chance. For example, a sequence is considered similar to another sequence if the smallest sum probability in comparison of the first sequence to the second sequence is less than about 1, preferably less than about 0.1, more preferably less than about 0.01, and most preferably less than about 0.001. Alternatively, the UWGCG Package provides the BESTFIT program which can be used to calculate identity (for example used on its default settings) (Devereux et al (1984) Nucleic Acids Research 12, 387-395).

### Conditioning regimen

The present invention provides an enzyme which inactivates serum IgG molecules in a subject for use in a conditioning regimen for the transplant of a cell, tissue or organ to a subject, comprising administering to the subject an enzyme which inactivates serum IgG molecules in the subject. The amount of said enzyme administered is preferably sufficient to inactivate all or substantially all IgG molecules present in the serum of the subject. If necessary, more than one IgG-inactivating enzyme can be administered in combination, including simultaneously or sequentially, in any order.

The term "serum IgG molecule(s)" or "IgG molecule(s) present in the serum" refers to any gamma immunoglobulin (IgG1, IgG2, IgG3 and IgG4) molecule which is present in human tissue or in circulation prior to a method of the invention being carried out. Such IgG molecules may have been produced endogenously from an individual's B-cells or may be exogenous gamma immunoglobulins which have been administered to a subject prior to the method of the invention being carried out - including any therapeutic IgG molecule of any origin. Inactivation of serum IgG typically means a reduction in the Fc receptor interaction of IgG molecules. The term "Fc receptor" refers to Fc gamma immunoglobulin receptors (FcyRs) which are present on cells. In humans, FcyR refers to one, some, or all of the family of receptors comprising FcyRI (CD64), FcyRIIA (CD32A), FcyRIIB (CD32B), FcyRIIC (CD32C), FcγRIIIA (CD16a) and FcyRIIIB (CD16b). As used herein, the term FcyR includes naturally occurring polymorphisms of FcyRI (CD64), FcyRIIA (CD32A), FcyRIIB (CD32B), FcγRIIC (CD32C), FcγRIIIA (CD16a) and FcγRIIIB (CD16b).

The enzyme used in the method of the invention may be any enzyme which inactivates serum IgG, but is typically an IgG cysteine protease which cleaves IgG such that the antigen binding domains and Fc interacting domains are separated from each other. In such cases, Fc receptor interaction of serum IgG molecules is reduced because the quantity of intact IgG molecules in the serum is reduced. As another example, the enzyme may be an IgG endoglycosidase which cleaves a glycan structure on the Fc interacting domain of IgG, particularly the N-linked bi-antennary glycan at position Asn-297 (Kabat numbering). This glycan structure has a critical role in Fc receptor binding and complement activation. Thus, when it is wholly or partially removed by a protein, this will lead to reduced Fc receptor binding or complement activation by an otherwise intact IgG molecule. Enzymes suitable for use in the conditioning regimen are discussed in more detail in subsequent sections below.

The enzyme is preferably administered by intravenous infusion, but may be administered by any suitable route including, for example, intradermal, subcutaneous, percutaneous, intramuscular, intra-arterial, intraperitoneal, intraarticular, intraosseous or other appropriate administration routes. The amount of the enzyme that is administered may be between 0.01 mg/kg BW and 2 mg/kg BW, between 0.05 and 1.5 mg/kg BW, between 0.1 mg/kg BW and 1 mg/kg BW, preferably between 0.15 mg/kg and 0.7 mg/kg BW
and most preferably between 0.2 mg/kg and 0.3 mg/kg BW, in particular 0.25 mg/kg BW. The enzyme may be administered on multiple occasions to the same subject, provided that the quantity of anti-drug antibody (ADA) in the serum of the subject which is capable of binding to the enzyme does not exceed a threshold determined by the clinician. The quantity of ADA in the serum of the subject which is capable of binding to the protease may be determined by any suitable method, such as an agent specific CAP FEIA (ImmunoCAP) test or a titre assay. If ADA in the subject exceed said threshold, the condition regimen may include administration of an alternative enzyme.

The conditioning regimen may additionally comprise one or more of:
(a) administration to the subject of a non-lethal dose of irradiation and/or any agent which depletes the subject's HPSC;
(b) administration of an agent to reduce the numbers and/or down-modulate the activity of lymphocytes in the subject, wherein said lymphocytes include:
   i. T cells; and/or
   ii. B cells (optionally including antibody-producing cells);
(c) administration any other agent or regimen which modulates (e.g. reduces) the activity of the immune system, e.g., inhibitors of complement, inhibitors of cytokines, inhibitors of innate immune cells, inducers of tolerance.

Step (a) typically involves administering a dose of radiation which is sufficient to partially or totally eradicate (or ablate) the bone marrow of the subject. Partial eradication is preferred since the side effects are typically less severe and also because it is desirable to retain some recipient bone marrow. The ablation of recipient bone marrow creates space in the bone marrow for engraftment of donor HSPCs, but also depletes lymphocytes in the subject and thus also reduces immune system activity in the same manner as step (b). As such, the conditioning regimen preferably includes at least (a), but most preferably includes at least (a) and (b). Alternatively, it may be preferred in step (a) to use an irradiation free approach to depletion of subject HSPCs, such as administration of anti-CD117 and/or anti-CD47. This will create space for engraftment of donor HSPCs, but without some of the undesirable side-effects of irradiation. In addition to HSPC depletion, the subject may also optionally receive an infusion of donor CD8-alpha cells, which may increase the frequency of stable chimerism in sensitized recipients. Donor T cell infusion may promote donor HSPC engraftment by reducing survival of host T cells.

Step (b) may be conducted by any suitable method and using any suitable agent. The same agent or combination of agents may be effective to reduce the numbers and/or down-modulate the activity of more than one type of lymphocyte. For example, preclinical studies in non-human primate models of transplantation in pre-sensitized recipients suggest that combining co-stimulation blockade by belatacept with plasma-cell depleting therapy by bortezomib may durably suppress DSA and decrease the risk of antibody mediated rejection.

Exemplary agents suitable for the depletion of T cells are known in the art and include anti-thymocyte globulin (ATG, such as rabbit or horse ATG); or a panel of antibodies including anti-CD4, anti-CD8, and anti-CD90; an anti-CD52 antibody (such as alemtuzumab); an anti-CD117 antibody; an anti-CD45 antibody; busulfan; cyclophosphamide; fludarabine; treosulfan; cyclosporin; tacrolimus; or an immunotoxin targeting T cells.

Exemplary agents suitable for depletion of B cells (optionally including plasma cells) are known in the art and include an anti-CD20 antibody (such as rituximab); an anti-CD19 antibody; bortezomib; fludarabine; cyclophosphamide; or an immunotoxin targeting B cells, such as an anti-CD20 immunotoxin (for example MT-3724).

An exemplary regimen including steps (a) and (b) is shown in the Examples. This includes the administration of a non-lethal dose of radiation, plus administration of a panel of antibodies including anti-CD4, anti-CD8, and anti-CD90 to deplete T cells, and of bortezomib and cyclophosphamide to deplete B cells (including antibody producing cells).

Steps (a) and (b) will typically be separated from each other, and where necessary also separated from the administration of the enzyme which inactivates serum IgG molecules in the subject, by whatever time interval is suitable for administration to have the desired effect. For example, where step (a) and/or (b) includes an antibody-based agent, it will be desirable for these steps to take place a sufficient time interval after the administration of the enzyme, such that the enzyme does not also inactivate the antibody-based agent of step (a) or (b). An exemplary time interval is illustrated in Example 2. Administration of rATG (or other antibody-based therapy) may be started as early as four days after administration of imlifidase. Alternatively, the enzyme may be added a suitable interval after the antibody-based agent, such that the antibody-based agent has already had its effect.

Administration of the enzyme which inactivates serum IgG molecules and steps (a) and (b) may take place at different times relative to a cell, tissue or organ transplant into the subject. For example, administration of the enzyme and steps (a) and (b) may all take place prior to a cell, tissue or organ transplant. Alternatively, administration of the enzyme and steps (a) and (b) may all take place after a cell, tissue or organ transplant. Alternatively, administration of the enzyme may take place before a cell, tissue or organ transplant with steps (a) and (b) afterwards. Alternatively, administration of the enzyme and (if present) step (a) may take place before a cell, tissue or organ transplant with step (b) afterwards. A typical method may include administration of the enzyme, followed by administration of a cell, tissue or organ transplant (such as a kidney transplant), followed by administration of ATG a suitable interval after the enzyme. For a transplant of HPSC (e.g. a bone marrow transplant) the order of steps may typically be an antibody-based agent of step (b), followed by the depletion of recipient HPSC of step (a), followed by the enzyme which inactivates serum IgG molecules in the subject, followed by the transplant.

### Method for inducing hematopoietic chimerism

The present invention provides an enzyme which inactivates serum IgG molecules in a subject for use in a method for the induction of hematopoietic chimerism in a subject, the method comprising conducting the conditioning regimen of the invention as defined in the claims and subsequently administering HSPC to the subject in an amount sufficient and under conditions suitable to induce hematopoietic chimerism in the subject. The method may alternatively be described as a method for the stable transplantation of HSPC. The HSPC may be autologous (the patient's own cells are used) or syngeneic (the cells are from a genetically identical twin), or they may allogeneic (the cells come from a separate, non-identical donor).

Immune complications which reduce the likelihood of successful engraftment of HSPC in the recipient are most significant for allogeneic cells and thus the method of the invention is of greatest benefit with such cells. However, immune complications can occur even with autologous cells if there is expression of a product to which the recipient has not previously been exposed. If an autologous cell has been genetically modified to express a gene therapy, the cell may be sufficiently altered to provoke an immune response. For example there may be an immune response to the expressed gene therapy product. Similar would apply if the HSPC has been genetically modified to express a different HLA type which is not matched to the HLA of the recipient. Therefore the HSPC are preferably allogeneic, or are genetically modified autologous or syngeneic cells. The HSPC are most preferably allogeneic. In a particularly preferred embodiment, the HSPC are from a donor who is also the donor of another organ or tissue which is to be transplanted into the recipient. That is, the same donor provides both the HSPC and the other cell, organ or tissue.

HSPC are found in the bone marrow of adults, especially in the pelvis, femur, and sternum. They are also found in umbilical cord blood and, in small numbers, in peripheral blood. HSPC may be harvested from these locations using any suitable technique established in the art.

For example, HSPC may be harvested from human bone marrow by aspirating directly from the centre of a bone of the donor with a large needle. The posterior iliac crest is the usual site of harvest. The technique is referred to as a bone marrow harvest and may be performed under local or general anesthesia. When the administered HSPC are derived from the bone marrow of the donor, the administration of HSPC may be described as a bone marrow transplant (BMT).

HSPC may be harvested from umbilical cord blood shortly after the birth of an infant. The umbilical cord is double-clamped from the umbilicus and transacted between clamps. The umbilical cord vein is then punctured under sterile conditions, and the blood flows freely by gravity into an anticoagulated sterile closed harvesting system, form which the HSPC may be isolated.

HSPC may be harvested from peripheral blood, typically by apheresis. However, because numbers of HSPC in peripheral blood are normally low, it is first necessary to mobilize HSPCs from the bone marrow. In a healthy donor, this can be achieved by administration of Granulocyte colony-stimulating factor (G-CSF). Alternative strategies may be required if the donor is not healthy. This may frequently be the case if the intended HSPC transplant is autologous.

HSPC are preferably used as quickly as possible after harvesting (that is fresh), but may be cryopreserved for storage prior to thawing for use with the invention. Cryopreservation typically includes volume depletion by removal of red cells and plasma. The quantity of stem cells in the harvest may be quantified, e.g. by flow cytometric analysis of a sample, to establish the proportion of cells which are positive for CD34 (a marker for stem cells).

The HSPC may be administered to the subject by any suitable method. A preferred method is infusion, typically through a central line. The patient may be kept in highly clean or sterile conditions, such as in a room with high-efficiency particulate air (HEPA) filters under positive pressure, before, during and after the infusion to reduce the risk of infection.

The method may be monitored to determine that the HSPC transplant has successfully resulted in hematopoietic chimerism. This is achieved by determining the proportion of donor-derived hematopoietic cells present in a blood sample taken from the subject after a particular time interval, typically 28 days after administration of the HSPC. For example, hematopoietic chimerism may be defined as achieved if at least 5% of the lymphocytes and/or myeloid cells in the sample are found to be donor-derived, preferably if at least 5% of the lymphocytes in the sample are found to be donor-derived. The chimerism is described as mixed if no more than 90% of the lymphocytes and/or myeloid cells in the sample are found to be donor-derived (that is at least 10% are still derived from the recipient), preferably if no more than 90% of the lymphocytes in the sample are found to be donor-derived (that is at least 10% of lymphocytes are still derived from the recipient). The chimerism may be described as total if 98% or more of the lymphocytes and/or myeloid cells in the sample are found to be donor-derived. Mixed chimerism is typically preferred for the methods of the invention, because the recipient will have a greater level of immunocompetence. However, full chimerism may be beneficial in some circumstances, for example in the treatment of cancers such as leukemia where the goal is to eliminate host cells with the potential to cause cancer recurrence, replacing them with the transplanted HSPC.

The proportion of donor and recipient derived cells in a sample may be determined by any suitable method in the art, such as flow cytometric analysis as described in the Examples. Real-time PCR may also be used. Other methods are discussed in Agrawal et al Bone Marrow Transplantation 2004 (34) p-12.

### Methods of treating or preventing a disease or condition

The present invention provides an enzyme which inactivates serum IgG molecules in a subject for use in a method for the prevention or treatment of a disease or condition in a subject, as defined in the claims. The method comprises inducing hematopoietic chimerism in a subject in accordance with the methods described above and as defined in the claims in order to improve the benefit to the subject of a therapy for the said disease or condition, thereby treating or preventing the disease or condition. Said therapy may be a cell, tissue or organ transplant, typically from the same donor as the HSPC. The cell, tissue or organ transplanted may be of any type, including kidney, liver, heart, pancreas, lung, small intestine, skin, blood vessels/vascular tissue, face, arm, trachea, parts of the eye, pancreatic islets, substantia nigra, bone marrow. The cell transplanted may be of any type, including the same HSPC as are used in the method itself, such that no additional therapy is required. The therapy may be a gene therapy administered using genetically modified HPSC.

Expressed another way, the invention also provides a method for the prevention or treatment of immune rejection of a cell, tissue or organ transplant, the method comprising inducing hematopoietic chimerism in the subject in accordance with the method of the invention as defined in the claims and administering a cell, tissue or organ transplant to the subject, optionally wherein said cell, tissue or organ is from the same donor as the HSPC. The cell, tissue or organ is typically administered after the induction of hematopoietic chimerism in the subject, but may be administered before. For example, if an organ is taken from a deceased donor it may be preferable to conduct the organ transplant first and subsequently induce hematopoietic chimerism using HSPC taken from the same deceased donor or a closely-matched donor. The cell, tissue or organ transplant may be of any type, including kidney, liver, heart, pancreas, lung, small intestine, skin, blood vessels/vascular tissue, face, arm, trachea, parts of the eye, pancreatic islets, substantia nigra, bone marrow. The cell transplanted may be of any type, including the same HSPC as are used to induce hematopoietic chimerism, such that no additional transplant is required.

The cell, tissue or organ to be transplanted may originate from a different species to the recipient, that is it may be a xenotransplant. Suitable species for xenotransplantation into human recipients may include pigs or non-human primates. In such cases the HSPC may be genetically modified to aid with tolerance to the transplant. The cell, tissue or organ that is a xenotranplant may also be genetically modified.

The subject to be treated may preferably be sensitized or highly sensitized. By "sensitized" it is meant that the subject has developed antibodies to human major histocompatibility (MHC) antigens (also referred to as human leukocyte antigens (HLA)). The anti-HLA antibodies originate from allogeneically sensitized B-cells and are usually present in patients that have previously been sensitized by blood transfusion, previous transplantation or pregnancy. Achieving hematopoietic chimerism in sensitized patients may reverse allosensitization, through the generation of specific tolerance in T and B cells, resulting in a reduction of donor specific immune responses such as DSA.

Whether or not a potential transplant recipient is sensitized may be determined by any suitable method. For example, a Panel Reactive Antibody (PRA) test may be used to determine if a recipient is sensitized. A PRA score >30% is typically taken to mean that the patient is "high immunologic risk" or "sensitized". Alternatively, a cross match test may be conducted, in which a sample of the potential transplant donor's blood is mixed with that of the intended recipient. A positive cross-match means that the recipient has antibodies which react to the donor sample, indicating that the recipient is sensitized and transplantation should not occur. Cross-match tests are typically conducted as a final check immediately prior to transplantation.

A method for the prevention or treatment of immune rejection of a cell, tissue or organ transplant comprises:
(i) conducting the conditioning regimen of the invention;
(ii) administering HSPC to the subject in an amount sufficient and under conditions suitable to induce hematopoietic chimerism in the subject.
The method may optionally also include (iii) administering to the subject a cell, tissue or organ transplant, which typically originates from the same donor as the HSPC. The HSPC administered in step (ii) may itself be the transplant, in which case no additional step (iii) is required. The method may be considered a method for the treatment of a disease or condition which is treated by the cell, tissue or organ transplant. For example, where the HSPC is itself the transplant, the method may be for the prevention or treatment of any disease or condition that is treated by HSPC transplant.

Diseases or conditions typically treated by HSPC transplant may be acquired or congenital. Acquired diseases or conditions that may be treated by HSPC transplant include:
- Hematological malignancies such as leukemias (for example Acute lymphoblastic leukemia (ALL), Acute myeloid leukemia (AML), Chronic lymphocytic leukemia (CLL), Chronic myelogenous leukemia (CML); lymphomas (for example Hodgkin's disease, Non-Hodgkin's lymphoma) and Myelomas (for example, Multiple myeloma (Kahler's disease)).
- Solid tumor cancers, such as Neuroblastoma, Desmoplastic small round cell tumor, Ewing's sarcoma, Choriocarcinoma.
- Hematologic diseases such as phagocyte disorders (for example Myelodysplasia); Anemias (for example Paroxysmal nocturnal hemoglobinuria (PNH; severe aplasia), Aplastic anemia, Acquired pure red cell aplasia); Myeloproliferative disorders (for example Polycythemia vera, Essential thrombocytosis, Myelofibrosis).
- Metabolic disorders such as amyloidosis (for example Amyloid light chain (AL) amyloidosis).
- Environmentally-induced diseases such as radiation poisoning.
- Viral diseases such as Human T-lymphotropic virus (HTLV) or Human Immunodeficiency Viruses (HIV).
- Autoimmune diseases, such as multiple sclerosis.
Congenital diseases or conditions that may be treated HSPC transplant include:
- Lysosomal storage disorders such as Lipidoses - disorders of lipid storage - (for example Neuronal ceroid lipofuscinoses, Infantile neuronal ceroid lipofuscinosis (INCL, Santavuori disease,), Jansky-Bielschowsky disease (late infantile neuronal ceroid lipofuscinosis)); Sphingolipidoses (for example Niemann-Pick disease, Gaucher disease); Leukodystrophies (for example Adrenoleukodystrophy, Metachromatic leukodystrophy, Krabbe disease (globoid cell leukodystrophy)); Mucopolysaccharidoses (for example Hurler syndrome (MPS I H, α-L-iduronidase deficiency), Scheie syndrome (MPS I S), Hurler-Scheie syndrome (MPS I H-S), Hunter syndrome (MPS II, iduronidase sulfate deficiency), Sanfilippo syndrome (MPS III), Morquio syndrome (MPS IV), Maroteaux-Lamy syndrome (MPS VI), Sly syndrome (MPS VII)); Glycoproteinoses (for example Mucolipidosis II (I-cell disease), Fucosidosis, Aspartylglucosaminuria, Alpha-mannosidosis; or Others (for example Wolman disease (acid lipase deficiency)
- Immunodeficiencies, such as T-cell deficiencies (for example Ataxia-telangiectasia, DiGeorge syndrome); Combined T- and B-cell deficiencies (for example Severe combined immunodeficiency (SCID), all types); Wiskott-Aldrich syndrome; Phagocyte disorders (for example Kostmann syndrome, Shwachman-Diamond syndrome); Immune dysregulation diseases (for example Griscelli syndrome, type II); Innate immune deficiencies (for example NF-Kappa-B Essential Modulator (NEMO) deficiency
- Hematologic diseases, such as Hemoglobinopathies (for example Sickle cell disease, β thalassemia major (Cooley's anemia)); Anemias (for example Aplastic anemia, Diamond-Blackfan anemia, Fanconi anemia); Cytopenias (for example Amegakaryocytic thrombocytopenia); and Hemophagocytic syndromes (for example Hemophagocytic lymphohistiocytosis (HLH)).

Where the HSPC are genetically modified to administer a gene therapy, the method of the invention may be for the prevention or treatment of the disease or condition to which said gene therapy is directed.

The invention also provides an enzyme which inactivates serum IgG molecules in a subject for use in a method for the prevention or treatment of a disease or condition, wherein the method is as described above.

The invention also provides the use of an enzyme which inactivates serum IgG molecules in a subject in the manufacture of a medicament, wherein the medicament is for the prevention or treatment of a disease or condition in a method as described above.

### Enzymes

### IgG cysteine proteases

The IgG cysteine protease for use with the invention is specific for IgG. In preferred embodiments, the protease for use in the methods of the invention is IdeS (Immunoglobulin G-degrading enzyme of *S. pyogenes*), otherwise known as imlifidase. IdeS is an extracellular cysteine protease produced by the human pathogen *S. pyogenes.* IdeS was originally isolated from a group A *Streptococcus* strain of serotype M1, but the *ides* gene has now been identified in all tested group A *Streptococcus* strains. IdeS has an extraordinarily high degree of substrate specificity, with its only identified substrate being IgG. IdeS catalyses a single proteolytic cleavage in the lower hinge region of the heavy chains of all subclasses of human IgG. IdeS also catalyses an equivalent cleavage of the heavy chains of some subclasses of IgG in various animals. IdeS efficiently cleaves IgG to Fc and F(ab')₂ fragments via a two-stage mechanism. In the first stage, one (first) heavy chain of IgG is cleaved to generate a single cleaved IgG (scIgG) molecule with a non-covalently bound Fc molecule. The scIgG molecule is effectively an intermediate product which retains the remaining (second) heavy chain of the original IgG molecule. In the second stage of the mechanism this second heavy chain is cleaved by IdeS to release a F(ab')₂ fragment and a homodimeric Fc fragment. These are the products generally observed under physiological conditions. Under reducing conditions the F(ab')₂ fragment may dissociate to two Fab fragments and the homodimeric Fc may dissociate into its component monomers. IdeS has been shown to be particularly effective at cleaving IgG in humans. The entire plasma IgG-pool is cleaved within minutes of dosing with IdeS, and IgG levels in blood remain low for more than a week until newly synthesized IgG appeared in plasma. This demonstrates that the entire extracellular IgG pool and not only the plasma pool (i.e. serum IgG molecules) is cleaved by IdeS (Winstedt et al; PloS One 2015; 10(7): e0132011).

SEQ ID NO: 1 is the full sequence of IdeS including the N terminal methionine and signal sequence. It is also available as NCBI Reference sequence no. WP_010922160.1. SEQ ID NO: 2 is the mature sequence of IdeS, lacking the N terminal methionine and signal sequence. It is also available as Genbank accession no. ADF13949.1.

In alternative embodiments, the protease for use with the invention is IdeZ, which is a IgG cysteine protease produced by *Streptococcus equi ssp. Zooepidemicus,* a bacterium predominantly found in horses. SEQ ID NO: 3 is the full sequence of IdeZ including N terminal methionine and signal sequence. It is also available as NCBI Reference sequence no. WP_014622780.1. SEQ ID NO: 4 is the mature sequence of IdeZ, lacking the N terminal methionine and signal sequence.

In alternative embodiments, the protease for use with the invention is a hybrid IdeS/Z, such as that of SEQ ID NO: 5. The N terminus is based on IdeZ lacking the N terminal methionine and signal sequence.

In preferred embodiments, the protease for use in the invention may comprise or consist of SEQ ID NO: 2, 4 or 5. Proteases for use in the invention may comprise an additional methionine (M) residue at the N terminus and/or a tag at the C terminus to assist with expression in and isolation from standard bacterial expression systems. Suitable tags include a histidine tag which may be joined directly to the C terminus of a polypeptide or joined indirectly by any suitable linker sequence, such as 3, 4 or 5 glycine residues. The histidine tag typically consists of six histidine residues, although it can be longer than this, typically up to 7, 8, 9, 10 or 20 amino acids or shorter, for example 5, 4, 3, 2 or 1 amino acids.

In further preferred embodiments, the protease for use in the invention may comprise, consist essentially, or consist of the sequence of any one of SEQ ID NOs: 6 to 25. These sequences represent IdeS and IdeZ polypeptides with increased protease activity and/or reduced immunogenicity. Each of SEQ ID NOs: 6 to 25 may optionally include an additional methionine at the N terminus and/or a histidine tag at the C terminus. The histidine tag preferably consists of six histidine residues. The histidine tag is preferably linked to the C terminus by a linker of 3x glycine or 5x glycine residues.

In further preferred embodiments, the protease for use in the invention may comprise, consist essentially, or consist of the sequence of any one of SEQ ID NOs: 56 to 69. These sequences represent IdeS polypeptides with increased protease activity and/or reduced immunogenicity. Each of SEQ ID NOs: 56 to 69 may optionally include an additional methionine at the N terminus and/or a histidine tag at the C terminus. The histidine tag preferably consists of six histidine residues. The histidine tag is preferably linked to the C terminus by a linker of 3x glycine or 5x glycine residues.

In further preferred embodiments, the protease for use in the invention may comprise, consist essentially, or consist of the sequence of any one of SEQ ID NOs: 6 to 25, optionally with up to 3 (such as 1, 2 or 3) amino acid substitutions. Each of SEQ ID NOs: 6 to 25 and variants thereof may optionally include an additional methionine at the N terminus and/or a histidine tag at the C terminus.

In further preferred embodiments, the protease for use in the invention may comprise, consist essentially, or consist of the sequence of any one of SEQ ID NOs: 56 to 69, optionally with up to 3 (such as 1, 2 or 3) amino acid substitutions. Each of SEQ ID NOs: 56 to 69 and variants thereof may optionally include an additional methionine at the N terminus and/or a histidine tag at the C terminus.

The polypeptide of the invention is typically at least 100, 150, 200, 250, 260, 270, 280, 290, 300 or 310 amino acids in length. The polypeptide of the invention is typically no larger than 400, 350, 340, 330, 320 or 315 amino acids in length. It will be appreciated that any of the above listed lower limits may be combined with any of the above listed upper limits to provide a range for the length the polypeptide of the invention. For example, the polypeptide may be 100 to 400 amino acids in length, or 250 to 350 amino acids in length. The polypeptide is preferably 290 to 320 amino acids in length, most preferably 300 to 315 amino acids in length.

The primary structure (amino acid sequence) of a protease of the invention is based on the primary structure of IdeS, IdeZ or IdeS/Z, specifically the amino acid sequence of SEQ ID NO: 2, 4 or 5, respectively. The sequence of a protease of the invention may comprise a variant of the amino acid sequence of SEQ ID NO: 2, 4 or 5, which is at least 80% identical to the amino acid sequence of SEQ ID NO: 2, 4 or 5. The variant sequence may be at least 80%, at least, 85%, preferably at least 90%, at least 95%, at least 98% or at least 99% identical to the sequence of SEQ ID NO: 2, 4 or 5. The variant may be identical to the sequence of SEQ ID NO: 2, 4 or 5 apart from the inclusion of one or more of the specific modifications identified in WO2016/128558 or WO2016/128559. Identity relative to the sequence of SEQ ID NO: 2, 4 or 5 can be measured over a region of at least 50, at least 100, at least 200, at least 300 or more contiguous amino acids of the sequence shown in SEQ ID NO: 2, 4 or 5, or more preferably over the full length of SEQ ID NO: 4 or 5.

The protease for use in the invention may be an IdeS, IdeZ or IdeS/Z polypeptide that comprises a variant of the amino acid sequence of SEQ ID NO: 2, 4 or 5 in which modifications, such as amino acid additions, deletions or substitutions are made relative to the sequence of SEQ ID NO: 2, 4 or 5. Such modifications are preferably conservative amino acid substitutions. Conservative substitutions replace amino acids with other amino acids of similar chemical structure, similar chemical properties or similar side-chain volume. The amino acids introduced may have similar polarity, hydrophilicity, hydrophobicity, basicity, acidity, neutrality or charge to the amino acids they replace. Alternatively, the conservative substitution may introduce another amino acid that is aromatic or aliphatic in the place of a pre-existing aromatic or aliphatic amino acid. Conservative amino acid changes are well-known in the art.

IgG cysteine protease activity may be assessed by any suitable method, for example by incubating a polypeptide with a sample containing IgG and determining the presence of IgG cleavage products. Suitable methods are described in the WO2016/128559. Suitable assays include an ELISA-based assay, such as that which is described in WO2016/128559. In such an assay, the wells of an assay plate will typically be coated with an antibody target, such as bovine serum albumin (BSA). Samples of the polypeptide to be tested are then added to the wells, followed by samples of target-specific antibody that is antibody specific for BSA in this example. The polypeptide and antibody are allowed to interact under conditions suitable for IgG cysteine protease activity. After a suitable interval, the assay plate will be washed and a detector antibody which specifically binds to the target-specific antibody will be added under conditions suitable for binding to the target-specific antibody. The detector antibody will bind to any intact target-specific antibody that has bound to the target in each well. After washing, the amount of detector antibody present in a well will be proportional to the amount of target-specific antibody bound to that well. The detector antibody may be conjugated directly or indirectly to a label or another reporter system (such as an enzyme), such that the amount of detector antibody remaining in each well can be determined. The higher the potency of the tested polypeptide that was in a well, the less intact target-specific antibody will remain and thus there will be less detector antibody. Typically, at least one well on a given assay plate will include IdeS instead of a polypeptide to be tested, so that the potency of the tested polypeptides may be directly compared to the potency of IdeS. IdeZ and IdeS/Z may also be included for comparison.

Other assays may determine the potency of a tested polypeptide by directly visualizing and/or quantifying the fragments of IgG which result from cleavage of IgG by a tested polypeptide. An assay of this type is also described in WO2016/128559. Such an assay will typically incubate a sample of IgG with a test polypeptide (or with one or more of IdeS, IdeZ and IdeS/Z as a control) at differing concentrations in a titration series. The products which result from incubation at each concentration are then separated using gel electrophoresis, for example by SDS-PAGE. Whole IgG and the fragments which result from cleavage of IgG can then be identified by size and quantified by the intensity of staining with a suitable dye. The greater the quantity of cleavage fragments, the greater the potency of a tested polypeptide at a given concentration. A polypeptide of the invention will typically produce detectable quantities of cleavage fragments at a lower concentration (a lower point in the titration series) than IdeZ and/or IdeS. This type of assay may also enable the identification of test polypeptides that are more effective at cleaving the first or the second heavy chain of an IgG molecule, as the quantities of the different fragments resulting from each cleavage event may also be determined. A polypeptide of the invention may be more effective at cleaving the first chain of an IgG molecule than the second, particularly when the IgG is an IgG2 isotype. A polypeptide of the invention may be more effective at cleaving IgG1 than IgG2.

### IgG endoglycosidases

The enzyme may have IgG endoglycosidase acitivty, preferably cleaving the glycan moiety at Asn-297 (Kabat numbering) in the Fc region of IgG. An example of such a protein is EndoS (Endoglycosidase of *S. pyogenes*). EndoS hydrolyzes the β-1,4-di-*N-*acetylchitobiose core of the asparagine-linked glycan of normally-glycosylated IgG. The mature sequence of EndoS is provided as SEQ ID NO: 90. The agent may be a protein comprising or consisting of the amino acid sequence of SEQ ID NO: 90, or may be a homologue thereof from an alternative bacterium, such as Streptococcus equi or Streptococcus zooepidemicus, or Corynebacterium pseudotuberculosis, Enterococcus faecalis, or Elizabethkingia meningoseptica. The agent may be CP40, EndoE, or EndoF₂.

Alternatively the agent may be a variant of the EndoS protein which comprises or consists of any amino acid sequence which has at least 80%, 85%, 90% or 95% identity with SEQ ID NO: 90 and has IgG endoglycosidase activity. A variant of the EndoS protein may comprise or consist of an amino acid sequence in which up to 1, 2, 3, 4, 5, 10, 20, 30, 40, 50, 60, 70, 80, 90, or more, amino acid substitutions, insertions or deletions have been made relative to the amino acid sequence of SEQ ID NO: 90, provided the variant has IgG endoglycosidase activity. Said amino acid substitutions are preferably conservative. Conservative substitutions are as defined in the preceding section.

Alternatively the agent may be a protein which comprises or consists of a fragment of SEQ ID NO: 90 and has IgG enodglycosidase activity, preferably wherein said fragment is 400 to 950, 500 to 950, 600 to 950, 700 to 950 or 800 to 950 amino acids in length. A preferred fragment consists of amino acids 1 to 409 of SEQ ID NO: 90, which corresponds to the enzymatically active α-domain of EndoS generated by cleavage by the streptococcal cysteine proteinase SpeB. The fragment may be created by the deletion of one or more amino acid residues of the amino acid sequence of SEQ ID NO: 90. Up to 1, 2, 3, 4, 5, 10, 20, 30, 40, 50, 100, 200, 300, 400, 500 or 550 residues may be deleted, or more. The deleted residues may be contiguous with other.

Any fragment or variant of SEQ ID NO: 90 preferably includes residues 191 to 199 of SEQ ID NO: 90, i.e. Leu-191, Asp-192, Gly-193, Leu-194, Asp-195, Val-196, Asp-197, Val-198 and Glu-199 of SEQ ID NO: 90. These amino acids constitute a perfect chitinase family 18 active site, ending with glutamic acid. The glutamic acid in the active site of chitinases is essential for enzymatic activity. Most preferably, therefore, a variant of SEQ ID NO: 90 contains Glu-199 of SEQ ID NO: 90. The variant of SEQ ID NO: 90 may contain residues 191 to 199 of SEQ ID NO: 90 having one or more conservative substitutions, provided that the variant contains Glu-199 of SEQ ID NO: 90.

### Production of polypeptides

The enzymes used in the invention are polypeptides and may be produced by any suitable means. For example, a polypeptide may be synthesised directly using standard techniques known in the art, such as Fmoc solid phase chemistry, Boc solid phase chemistry or by solution phase peptide synthesis. Alternatively, a polypeptide may be produced by transforming a cell, typically a bacterial cell, with a nucleic acid molecule or vector which encodes said polypeptide. Production of enzyme polypeptides by expression in bacterial host cells is described and exemplified in WO2016/128558 and WO2016/128559.

### Compositions and formulations comprising polypeptides

The present invention also provides compositions comprising an enzyme for use in the invention as defined in the claims. For example, the invention provides a composition comprising one or more polypeptides, and at least one pharmaceutically acceptable carrier or diluent. The carrier(s) must be 'acceptable' in the sense of being compatible with the other ingredients of the composition and not deleterious to a subject to which the composition is administered. Typically, carriers and the final composition are sterile and pyrogen free.

Formulation of a suitable composition can be carried out using standard pharmaceutical formulation chemistries and methodologies all of which are readily available to the reasonably skilled artisan. For example, the enzyme can be combined with one or more pharmaceutically acceptable excipients or vehicles. Auxiliary substances, such as wetting or emulsifying agents, pH buffering substances, reducing agents and the like, may be present in the excipient or vehicle. Suitable reducing agents include cysteine, thioglycerol, thioredoxin, glutathione and the like. Excipients, vehicles and auxiliary substances are generally pharmaceutical agents that do not induce an immune response in the individual receiving the composition, and which may be administered without undue toxicity. Pharmaceutically acceptable excipients include, but are not limited to, liquids such as water, saline, polyethylene glycol, hyaluronic acid, glycerol, thioglycerol and ethanol. Pharmaceutically acceptable salts can also be included therein, for example, mineral acid salts such as hydrochlorides, hydrobromides, phosphates, sulfates, and the like; and the salts of organic acids such as acetates, propionates, malonates, benzoates, and the like. A thorough discussion of pharmaceutically acceptable excipients, vehicles and auxiliary substances is available in Remington's Pharmaceutical Sciences (Mack Pub. Co., N.J. 1991).

Such compositions may be prepared, packaged, or sold in a form suitable for bolus administration or for continuous administration. Injectable compositions may be prepared, packaged, or sold in unit dosage form, such as in ampoules or in multi-dose containers containing a preservative. Compositions include, but are not limited to, suspensions, solutions, emulsions in oily or aqueous vehicles, pastes, and implantable sustained-release or biodegradable formulations. Such compositions may further comprise one or more additional ingredients including, but not limited to, suspending, stabilizing, or dispersing agents. In one embodiment of a composition for parenteral administration, the active ingredient is provided in dry (for e.g., a powder or granules) form for reconstitution with a suitable vehicle (e. g., sterile pyrogen-free water) prior to parenteral administration of the reconstituted composition. The compositions may be prepared, packaged, or sold in the form of a sterile injectable aqueous or oily suspension or solution. This suspension or solution may be formulated according to the known art, and may comprise, in addition to the active ingredient, additional ingredients such as the dispersing agents, wetting agents, or suspending agents described herein. Such sterile injectable formulations may be prepared using a non-toxic parenterally-acceptable diluent or solvent, such as water or 1,3-butane diol, for example. Other acceptable diluents and solvents include, but are not limited to, Ringer's solution, isotonic sodium chloride solution, and fixed oils such as synthetic mono-or di-glycerides.

Other parentally-administrable compositions which are useful include those which comprise the active ingredient in microcrystalline form, in a liposomal preparation, or as a component of a biodegradable polymer systems. Compositions for sustained release or implantation may comprise pharmaceutically acceptable polymeric or hydrophobic materials such as an emulsion, an ion exchange resin, a sparingly soluble polymer, or a sparingly soluble salt. The compositions may be suitable for administration by any suitable route including, for example, intradermal, subcutaneous, percutaneous, intramuscular, intra-arterial, intraperitoneal, intraarticular, intraosseous or other appropriate administration routes. Preferred compositions are suitable for administration by intravenous infusion.

### Kits

A kit for carrying out the methods described herein is also described. The kit may include an enzyme or a composition comprising an enzyme, as described above. The kit may include means for administering the enzyme or composition to a subject. The kit may include instructions for use of the various components in any method as described herein.

### EXAMPLES

Unless indicated otherwise, the methods used are standard biochemistry and molecular biology techniques. Examples of suitable methodology textbooks include Sambrook et al., Molecular Cloning, A Laboratory Manual (1989) and Ausubel et al., Current Protocols in Molecular Biology (1995), John Wiley and Sons, Inc.

### EXAMPLE 1

### Introduction

Imlifidase cleaves all human IgG subclasses, but only cleaves mouse IgG2c and IgG3, and not mouse IgG1 and IgG2b. Interestingly, EndoS has been shown to reduce complement- and FcyR-mediated functions of mouse IgG1 and IgG2b. However, EndoS-treated mouse IgG2a and IgG2c have been shown to maintain cytolytic activity via FcyR but IgG2c has also been shown to lose some binding affinity depending on the conditions. Therefore, for the purposes of the animal models used in the following experiments, a combination of imlifidase and EndoS has been used to ensure the greatest effect on serum IgG in the murine subjects. It is expected that either imlifidase or EndoS alone (or another protease or endoglycosidase of comparable specificity/activity) will be sufficient to achieve comparable effects in human subjects.

The following experiments use a stringent model of sensitized NOD recipients that are resistant to irradiation and tolerance induction. The experiments demonstrate that a combined approach that includes both imlifidase and EndoS permits the generation of mixed hematopoietic chimerism in these mice.

### Materials and Methods

### Animals

Adult NOD/ShiLtJ (H-2^{g7}; termed NOD), FVB/NJ (H-2^{q}; termed FVB), C57BL/6J (H-2^{b}; termed B6.CD45.2), B6.SJL-Ptprc a Pepcb./Boy (H-2^{b}, term B6.CD45.1), B6.NOD-(D17Mit21-D17Mit10) (H-2^{g7}; termed B6.H-2^{g7}), NOD.B10Sn-H2^{b}/J (H-2^{b}; termed NOD.H-2^{b}) mice were purchased from the Jackson Laboratory (Bar Harbor, ME, USA), bred and housed in a specific pathogen-free facility at the University of Alberta. All care and handling of animals were conducted in accordance with the guidelines of the Canadian Council on Animal Care. All NOD mice used for chimerism induction were females at 8 to 10 weeks of age.

### Reagents for in vivo experiments

Imlifidase and EndoS were provided by Hansa Biopharma AB (Lund, Sweden) and used with permission. Anti-CD4 (clone Gk1.5, rat IgG_{2b}), anti-CD90 (clone YTS154, rat IgG_{2b}), anti-CD8α (clone YTS169.4, rat IgG_{2b}), and anti-MHC-I H-2K^{b} (clone B8.24.3, mouse IgG_{2b}) mAbs were generated in house. The YTS 169.4 anti-mouse CD8α mAb producing cells were developed by Prof. H Waldmann and Dr. SP Cobbold (Department of Pathology, University of Cambridge) and obtained via Cambridge Enterprise Limited (Hauser Forum, 3 Charles Babbage Road, Cambridge CB3 0GT). Cyclophosphamide (29875) and bortezomib (A2614) were purchased from Sigma (MO, USA) and ApexBio (TX, USA), respectively.

### In vivo EndoS mediated monoclonal DSA inhibition assay

NOD or B6.H-2^{g7} mice were i.v. injected with vehicle, anti-MHC-I H-2K^{b} (10µg) alone, or a mixture of EndoS (30µg) and anti-MHC-I H-2K^{b} (10µg or 100µg) as a pretreatment. EndoS and anti-H-2K^{b} were mixed right before injection. Four hours after this pretreatment, five million cells of a 1:1 mixture of carboxyfluorescein succinimidyl ester (CFSE) labeled NOD and cell trace violet dye (CTV) labeled B6 bone marrow cells (BMC) were i.v. injected into the pre-treated NOD mice. Similarly, a 1:1 mixture of CFSE labeled B6.H-2^{g7} and CTV labeled NOD.H-2^{b} BMC were injected into pre-treated B6.H-2^{g7} mice. Blood was collected at 1, 2, and 3 hours post cell administration and analyzed by flow cytometry. Splenocytes and BMC from one hind limb were collected from each mouse and analyzed at four hours post BMC injection.

### Serum DSA detection assay

NOD mice were sensitized by i.p. administration of 20×10⁶ FVB splenocytes. Sera were collected prior to and at 4 to 6 weeks post sensitization as well as at 4 hours post imlifidase and EndoS treatment. FVB splenocytes (2x10⁵) were treated with FcR blockade (anti-mouse CD16/CD32 rat IgG_{2b} antibodies, clone 2.4G2, BE0307, Bio X cell) for 5 minutes, followed by incubation with a titrated amount of sera in 100µL for 30 minutes. Cells were washed twice and incubated with fluorochrome conjugated secondary antibodies in 100µL for 30 minutes. The following secondary antibodies were used: FITC conjugated F(ab')₂ fragment from rabbit anti-mouse IgG Fc antibody (1:200, 315-096-046, Jackson ImmunoResearch), APC conjugated goat anti-mouse IgG₁ Fc antibody (1:100, 115-135-205, Jackson ImmunoResearch), and FITC conjugated goat anti-mouse IgG₃ Fc antibody (1:100, 115-095-209, Jackson ImmunoResearch). Cells were washed twice and analyzed by flow cytometry. HBSS with 2% FBS was used for cell washes and reconstitution.

### BMT protocol and definition of chimerism

To determine the short-term survival of donor BMC in sensitized recipients, NOD mice that had been sensitized to B6.CD45.1 splenocytes were T cell-depleted (anti-CD4, 0.25mg, anti-CD8, 0.25mg, anti-CD90 0.3mg, i.p.) two days prior to BMT and i.v. injected with EndoS and imlifidase 4 hours prior to BMT (80×10⁶ B6.CD45.2 BMC via i.v. injection). Splenocytes and BMC were analyzed at 4 hours post BMC injection.

For long-term chimerism induction, NOD mice that had been sensitized to FVB splenocytes were treated with imlifidase and EndoS i.v. on day -6 with respect to the date of BMT. Cyclophosphamide (150mg/kg, i.p. or i.v.) and bortezomib (1mg/kg, i.v.) were given on day -4. T cell-depleting antibodies were administered i.p. on day -2, 2, 6, 11, and 16. A repeated dose of imlifidase and EndoS and 6 Gy total body irradiation (TBI, Gammacell 1000 Elite) was given at four hours prior to BMT on day 0. FVB bone marrow cells (80×10⁶) were given intravenously (i.v.) via the lateral tail vein on day 0. In experiments determining the effects of cyclophosphamide and bortezomib on sensitized recipients prior to BMT, a lower dose (20×10⁶) of bone marrow cells was given to limit potential adsorption of DSA on donor bone marrow cells. Peripheral blood was collected for flow cytometry analysis at the indicated time points. For long-term chimerism, recipients were considered chimeric when at least 5% of MHC-I⁺ cells in the lymphocyte gate were donor-derived at day 28 post-BMT.

### Antibodies and flow cytometry

Fluorochrome-labeled antibodies against mouse H-2K^{d} (SF1-1.1.1), H-2K^{q} (KH114), H-2K^{b} (AF6-88.5), CD45.2 (104), CD19 (6D5), CD138 (281-2), B220 (RA3-6B2), TCRβ (H57-597), CD4 (RM4-5 or RM4-4), CD8β (H35-17.2), CD11b (M1/70), CD11c (N418), CD49b (DX5), CD122 (TM-β1), were purchased from BD Pharmingen (CA, USA), BioLegend (CA, USA) or Thermo Fisher Scientific (CA, USA). An LSR II (Becton Dickson, CA, USA) flow cytometer was used for data acquisition, and data analysis was performed using FlowJo (Treestar software, OR, USA).

### Statistical analysis

Mann-Whitney U test, Ratio paired t-test, one-way ANOVA with Holm-Sidak's multiple comparison test, and Fisher's exact test were used where appropriate, as indicated. All statistical analyses were done using Prism (GraphPad Software, CA, USA).

### Results

### EndoS inhibits the monoclonal DSA mediated killing of donor BMC

To evaluate the effect of EndoS on inhibiting the antibody-mediated killing of donor BMC, DSA passive transfer experiments were performed. Of all DSA, anti-donor MHC or HLA antibodies are of most importance in the clinic. Therefore, naive NOD mice were injected with mouse IgG_{2b} antibodies targeting MHC-I K^{b} expressing cells, treated with EndoS or left untreated, and thereafter subjected to bone marrow transfer from B6 mice.

As shown in Fig 1A-B, in NOD recipients given a single dose of 10µg anti-K^{b} mAb, the ratios of B6 to NOD cells in blood at one hour post-BMT were significantly increased in mice treated with EndoS as compared to those that did not receive enzyme treatment. This difference in ratio of B6 to NOD cells in blood between the two groups remained stable at two and three hours post-BMT. Similarly, mice given 100µg anti-K^{b} mAb with EndoS led to an increased ratio of B6 to NOD cells in the blood at one and two hours compared with treatment with 100µg anti-K^{b} mAb alone. However, the increased ratio did not last to three hours, suggesting that residual mAb effector function accumulated over time. At four hours post-BMT, a significant increase in the ratio of B6 to NOD cells in both BM and spleen was also observed in mice treated with EndoS and 10µg anti-K^{b} mAb as compared to those that received 10µg anti-K^{b} mAb only.

Of note, NOD mice lack hemolytic complement C5, which is essential for complement dependent cytotoxicity and is not genetically linked with MHC genes. Thus, the effect of DSA in NOD mice may be decreased compared with complement sufficient hosts. The role of EndoS on DSA in complement sufficient hosts was therefore also examined. NOD MHC congenic B6.H-2^{g7} mice were used as recipients. EndoS improved the ratios of donor to recipient cells to a similar extent in B6.H-2^{g7} mice as compared to NOD hosts (Fig 1C-D).

In brief, EndoS improved survival of donor cells in the presence of anti-MHC antibodies whether or not the recipients were complement-sufficient, suggesting an effect on other mechanisms of depletion, e.g., FcgR-mediated), at least in this model system.

### EndoS improves survival of donor BMC in presensitized recipients

Next, it was investigated if EndoS could improve donor BMC survival in allo-sensitized recipients that had a diversified antibody repertoire against donor antigens. In order to test this, EndoS was used in combination with imlifidase. Imlifidase cleaves murine IgG_{2c} and IgG₃ but is not able to cut murine IgG₁ and IgG_{2b}. Therefore, EndoS was co-administered to attenuate the effector function of the murine IgG isotypes that are not cleaved by imlifidase. As shown in Fig 2A, imlifidase and EndoS together led to a significant reduction of DSA-IgG in NOD mice that had been sensitized to FVB splenocytes. The decline in IgG-targeting of donor cells was likely due to imlifidase, and not EndoS, since deglycosylation still allows the Fc-specific detection antibody to bind. The differential sensitivity for murine IgG isotypes is also illustrated by the approximately 80% reduction of DSA-IgG₃ (Fig 2C), a subclass that is cleaved by imlifidase, whereas no change in the level of DSA-IgG₁ (Fig 2B) was seen. While the degradation of IgG₃ by imlifidase only caused a moderate reduction of intact IgG, EndoS could further contribute to the reduction of DSA-IgG effector functions through the deglycosylation of imlifidase resistant IgG molecules. The combination of both enzymes allowed the analysis donor cell survival in sensitized recipients with polyclonal DSA.
In addition to DSA, primed donor antigen-specific cytotoxic T cells may contribute to the rapid killing of donor BMC. Therefore, CD45.1 NOD recipients that had been sensitized with congenic B6.CD45.1 splenocytes were T cell-depleted two days before imlifidase and EndoS treatment in order to avoid the acute cytotoxic effect mediated by sensitized T cells (Fig 2D). Over 95% of T cells in the peripheral blood were depleted in the recipients at two days after giving T cell-depleting mAbs (data not shown). Here, the CD45.1/2 system was used to assist the identification of surviving donor BMC, the MHC staining on which may be interfered with by DSA. As shown in Fig 2E-F, B6.CD45.2 donor cells were almost completely eliminated at four hours post-BMT in sensitized NOD mice when given vehicle control (BM 0.22% and spleen 0.27%) or only imlifidase (BM 0.15% and spleen 0.46%). In contrast, close to 0.5% of BMC and around 1.5% of splenocytes in sensitized NOD mice treated with EndoS and imlifidase were from the B6.CD45.2 donor. Thus, administration of imlifidase and EndoS four hours prior to BMT rescued a significant proportion of donor BMC in allo-sensitized recipients as compared to sensitized recipients treated with vehicle or imlifidase alone (Fig 2F). Interestingly, the majority of residual donor cells in recipients treated with imlifidase and EndoS demonstrated low MHC-I K^{b} staining, suggesting donor MHC epitopes were blocked by either de-glycosylated DSA or F(ab')₂ of DSA (Fig 2E). Alternatively, the surviving donor cells may have been those that expressed less MHC class I.

Taken together, these data indicated that the combination of imlifidase and EndoS improved the donor BMC survival in allosensitized recipients. In other words, inactivation of substantially all serum IgG improved donor BMS survival in allo-sensitized recipients.

### Bortezomib and cyclophosphamide treatment prior to BMT reduced B cells in BM

In addition to imlifidase and/or EndoS for BMT, methods that also reduce DSA-producing cells may provide a longer window of the low DSA environment for the continuous survival and further development of donor cells post BMT. In an attempt to reduce existing plasma cells and B cells that can differentiate into plasma cells after BMT, bortezomib was employed to deplete antibody-producing cells and cyclophosphamide to reduce B cells prior to BMT in sensitized mice (Fig 3A). The combination of bortezomib and cyclophosphamide (CyBor) has been used in patients with non-transplant eligible multiple myeloma and for prevention of graft-versus-host disease (GVHD) post allogeneic BMT, but rarely used for the purpose of DSA desensitization.

At five days after BMT, the cellularity of BMC in the BM did not differ between groups. Interestingly, the overall number of splenocytes increased in the group of mice pretreated with CyBor. However, compared to the vehicle group, BM CD19⁺ B cells, CD19⁻CD138⁺B220⁺ plasmablasts, and CD19⁻CD138⁺B220⁻ plasma cells were significantly reduced in mice treated with CyBor (Fig 3B). In contrast to the reduction of B cells in the BM, the reduction of splenic CD19⁺ B cells was not significant at the time examined in the CyBor treated group. Moreover, there were significant increases of CD19⁻CD138⁺B220⁺ plasmablasts and CD19⁻CD138⁺B220⁻ plasma cells in the spleens from CyBor treated mice (Fig 3C).

It was then examined whether the CyBor treatment prevented increased DSA formation stimulated by the BMC injection. As shown in Fig 3D, DSA levels increased substantially in two of five mice in the control group and two of five mice in the CyBor treated group, suggesting that CyBor was not able to decrease DSA levels. However, when percentile changes of DSA levels five days after BMT (nine days post-CyBor) were compared, the increases of DSA tended to be less in mice treated with CyBor, suggesting that CyBor treatment prior to BMT may inhibit the increase of DSA stimulated by BMC injection (Fig 3E).

In summary, these data showed the effect of CyBor in reducing B cell numbers was pronounced in BM and CyBor may limit the increase in DSA caused by the BMC injection.

### Engraftment is achievable in presensitized recipients with combination of Imlifidase, EndoS, T cell depletion, and CyBor

With the data above, it was hypothesized that imlifidase and EndoS in combination with T cell depletion antibodies and bone marrow plasma cell depletion by CyBor, together with a non-lethal dose of irradiation, and a large dose of BMC would allow engraftment of donor cells in presensitized recipients. It was explored if such protocol would induce chimerism in NOD mice as well as in B6.H-2^{g7} mice, which are MHC matched with NOD but are not resistant to chimerism induction. Recipient mice were sensitized with FVB cells four weeks prior to the chimerism induction. Naive and primed recipients were given the same conditioning protocol, as indicated in the methods section and Fig 4A.

As expected, while all naive mice became nearly fully chimeric with FVB cells at four weeks post-BMT, donor cells were rejected in primed mice that were not treated with imlifidase and EndoS. As shown in Fig 4B, donor cells were not detectable even at two days post-BMT in sensitized NOD mice that did not receive enzymes. In contrast, donor cells were more than five percent on day 4 or 9 after BMT in five out of seven sensitized NOD recipients given enzyme treatment. Furthermore, in four enzyme-treated sensitized NOD mice, chimerism levels increased steadily to over 50 percent on day 16 post-BMT. Eventually, five of the eight presensitized NOD and B6.H-2^{g7} mice were chimeric with donor cells at four weeks post-BMT, with two primed NOD mice being stable mixed chimeras with multiple lineages of donor cells in the periphery (Table 1 and Fig 4C). No sign of GVHD was observed in any chimeras. In an attempt to simplify this protocol by eliminating either cyclophosphamide or bortezomib, it appeared that both of them were essential for the success of the current protocol for inducing chimerism in sensitized recipients (Table 1).

In summary, a combination of imlifidase and EndoS (i.e. the inactivation of substantially all serum IgG) enables donor BMC engraftment in presensitized recipient mice when combined with CyBor and standard conditioning agents.

**Table 1 EndoS-imlifidase allows hematopoietic chimerism in pre-sensitized recipients**

| **Treatment group** | | | **Engraftment^{†}** | **Chimerism levels^{#}** |
|---|---|---|---|---|
| Not primed | | | | |
| | CyBor | | 6/6^{†} | >90% |
| Primed | | | | |
| | CyBor | | 0/7^{‡} | |
| | CyBor-EndoS-imlifidase* | | 5/8 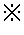 | 98%, 85%, 57%, 20%, 9% |
| | | Cy-EndoS-imlifidase | 0/2^{¤} | |
| | | Bor-EndoS-imlifidase | 0/3^{¤} | |

| | | | | |
|---|---|---|---|---|
| See figure legend of Fig 4 for details of chimerism induction protocol. ^{†}represents B6.H-2^{g7} (n=2) and NOD recipients (n=4). ^{‡} represents B6.H-2^{g7} (n=2) and NOD recipients (n=5). *represents one B6.H-2^{g7} (n=1) and NOD recipients (n=7). ^{¤} represents NOD recipients. ^{#}Shown are chimerism levels at four weeks post BMT. *p<0.05 by two-sided Fisher's exact test when compared to "CyBor" primed group. | | | | |

### Discussion

DSA is a major obstacle for allogeneic BMT in sensitized recipients. Previous work showed that imlifidase can be used for eliminating / reducing DSA and EndoS can inhibit IgG-mediated cytotoxicity in various models, but neither enzyme has been used in the context of HSPC transplant / bone marrow transplant, where the high expression of MHC on bone marrow derived cells may increase sensitivity to remaining functional DSA.

Previous results from recent clinical trials for kidney transplantation in sensitized recipients, taken together with these experiments show that imlifidase can indeed be used to condition human patients to receive HSPC transplant / bone marrow transplant. The current study also shows that EndoS can be used in this context. It was found that EndoS alone improved survival of donor cells in the presence of DSA *in vivo.* Considering that EndoS-treated IgG reduces the ability to fix complement, as reported by Maria Allhorn and Mattias Collin, our finding that EndoS improved the survival of donor cells to a similar extent in B6.H-2^{g7} and NOD suggested that additional mechanisms such as FcgRs were a major mediator of the pathogenicity of DSA in this BMT-model. The differences between NOD and B6.H-2^{g7} mice given a low or high dose of monoclonal DSA and EndoS indicate that the non-MHC genes may have an impact on the efficacy of EndoS in different individuals. This difference between NOD and B6.H-2^{g7} may be attributable to the different binding capacities of IgG_{2b} with various Fc receptors in mice on the NOD and B6 background. FcR polymorphisms may be important as well. The results also suggest that the effects of EndoS are more potent on lower titer DSA.

It was found that the combination of imlifidase and EndoS improved the survival of donor BMC and allowed donor chimerism in sensitized mice that had been conditioned with T cell depletion, CyBor, and sublethal irradiation. In the tested protocol, the effect of T cell depletion in the periphery was not affected by EndoS. This suggests that with appropriately designed timing, enzyme depletion of serum IgG can be used together with antibody-based products like IVIG and B cell depletion antibodies such as rituximab. In other words, enzymes could be used to inactivate DSA without negatively affecting the effector functions of IgG-based biologics, provided the timing of administration of each is carefully selected.

With regard to the use of cyclophosphamide and bortezomib, both have immune modulatory effects other than targeting B cells or plasma cells. For example, cyclophosphamide can facilitate the chimerism induction in sensitized recipients by reducing memory T cells. As for bortezomib, the finding is consistent with the published data showing the compensatory increase of splenic B cells after bortezomib treatment, which in turn resulted in humoral compensation. However, whether or not this increase of splenic B cells after BMT is accompanied by a rebound of DSA in the current study remains unknown. Importantly, T cell depletion employed in this protocol may potentially inhibit the recovery and maturation of both naïve and memory B cells, and the generation of de novo DSA.

Lastly, the findings of this study have to be considered in light of some limitations. Although imlifidase cleaves all the human IgG subclasses, it only cleaves two subclasses of mouse IgG, and IgM is not affected. Although IgM DSA levels are low compared to IgG, they may have reduced the levels of chimerism that were observed. In the clinic, IgM DSA could be removed by plasmapheresis. In order to achieve maximum effect on DSA in mice, it was necessary to combine EndoS and imlifidase. It has been shown that imlifidase temporally inhibits the activation of memory B cells by cleavage of membrane-bound BCR *in vitro,* which may contribute to the success of chimerism. However imlifidase only cleaves mouse IgG_{2c} and IgG₃, so the effect of imlifidase on mouse IgG was not complete in this model (Fig 2A). A protocol with imlifidase as only the desensitizing agent will be more efficient in humans where imlifidase completely removes / inactivates all extracellular IgG, and so completely inactivates the IgG DSA pool. Thus, these findings may underestimate the potential for these enzymes in the clinical setting.

The second limitation concerns the toxicity of the chimerism induction protocol. However, the current study is a proof of principle study showing that modulating IgG Fc can be strategically useful for BMT in sensitized recipients. Furthermore, EndoS or imlifidase can be used in combination with other desensitization methods. Currently, it is not known whether the enzyme-mediated blocking of DSA prevents a rebound in antibody. Perhaps maintaining a certain level of DSA while blocking DSA function, i.e. de-glycosylation of IgG Fc, may have less potential to trigger a rebound than complete removal of the DSA. These experiments employed a short time frame for repeated enzyme injection (6 days between injections) in order to avoid reduced activity as a result of host anti-enzyme antibody production. The greater efficacy of imlifidase in the human setting may allow the enzymes to be given separately (e.g. imlifidase followed by EndoS), alleviating any concern that may arise with anti-enzyme antibodies.

Finally, it can be concluded that the combination of imlifidase and EndoS (that is the enzymatic inactivation of substantially all serum IgG) can be used for inducing donor chimerism in allo-sensitized recipient mice in combination with other desensitization strategies.

### Example 2 - optimal spacing of imlifidase and antibody-based therapies

### Background

Imlifidase (conditionally authorised in the EU for kidney transplant desensitization) is a cysteine protease which cleaves all subclasses of human and rabbit IgG to a F(ab')₂ fragment and a dimeric Fc fragment. Rabbit anti-thymocyte globulin (rATG) is a depleting antibody therapy approved for induction in kidney transplantation (it effects a large reduction in circulating T-lymphocytes). Antibody-based therapies such as rATG may be inactivated if given with imlifidase. The purpose of this study was to investigate the earliest time point to start rATG treatment while avoiding most of the cleavage activity of remaining imlifidase.

### Methods

The cleavage pattern of rATG was investigated with sera from healthy subjects (n=11) treated with 0.25 mg/kg imlifidase (EudraCT number: 2019-002770-31). Serum samples were incubated with a fixed, clinically relevant, concentration of 50 µg/mL rATG (commonly observed after a dose of 1.5 mg/kg), for 2 hours at 37°C. Serum samples were collected pre-imlifidase through 14 days post-imlifidase and were analyzed using SDS-PAGE and Western blot, developed with a goat anti-rabbit IgG, F(ab')₂ specific antibody to evaluate the cleavage of rATG. Imlifidase concentration was analyzed using a validated electroluminescence immunoassay based on MSD technology.

### Results

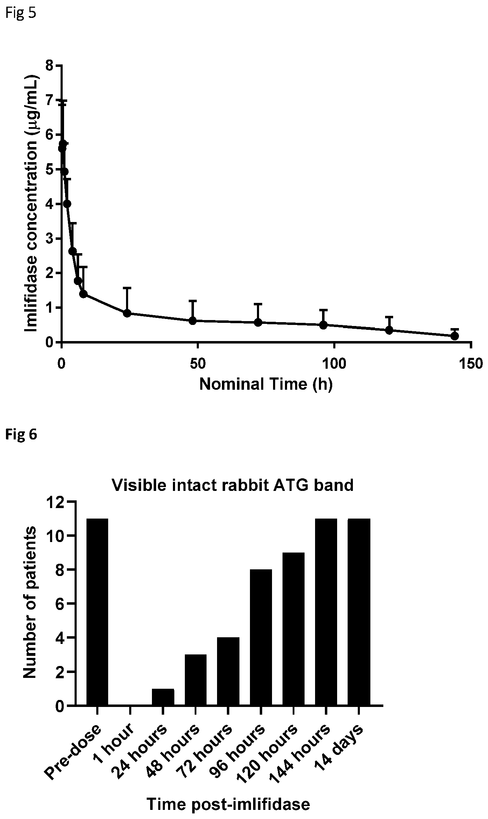

The imlifidase serum concentration in the subjects declined rapidly and at 96 hours the mean concentration was 0.5 µg/mL, though with a large individual variation, <0.1-1.8 µg/mL (Figure 5). At this timepoint the level of imlifidase activity had decreased sufficiently to avoid complete cleavage of rATG in 8 of 11 subjects (Figure 6).

### Conclusions

rATG may be started as early as 4 days post-imlifidase, taking into consideration that a portion of the first rATG administration may be cleaved in some patients. However, since the rATG dose is high and administration repeated for several days, this cleavage at the start of therapy is not anticipated to have a negative overall effect on the rATG treatment efficacy.

### Example 3 - enhanced specificity and reduced toxicity for mixed chimerism protocol

Stepwise changes will be introduced into the mixed chimerism protocol set out in Example 1, aimed at increasing the specificity and reducing the potential toxicity of the approach, and thus achieving a greater potential for clinical translation. In particular:
(i) an infusion of donor CD8-alpha cells will be administered to increase the frequency of stable chimerism in sensitized recipients. Donor T cell infusion may promote BMT engraftment by reducing survival of host T cells.
(ii) Together with elimination of DSA by enzyme (IdeS and/or EndoS) and maximal T and NK cell depletion, anti-CD117 / anti-CD47 will be administered. This will allow for the first irradiation free, non-myeloablative chimerism protocol for pre-sensitized recipients. The anti-CD117/anti-CD47 antibodies help to deplete host HSCs.

## Claims

1. An enzyme which inactivates serum IgG molecules in a subject for use in a conditioning regimen for the transplant of hematopoietic stem and progenitor cells (HSPC) to a subject, comprising administering the enzyme to the subject.

2. The enzyme for use of claim 1, wherein the amount of said enzyme administered is sufficient to inactivate all or substantially all IgG molecules present in the serum of the subject.

3. The enzyme for use of any preceding claim, wherein the enzyme is an IgG cysteine protease or an IgG endoglycosidase.

4. The enzyme for use of claim 3, wherein:
(i) the IgG cysteine protease is from a Streptococcus bacterium such as Streptococcus pyogenes, optionally wherein said enzyme is a IdeS, IdeZ or MAC2 polypeptide, or
(ii) the IgG endoglycosidase is from a Streptococcus bacterium, such as Streptococcus pyogenes, Streptococcus equi or Streptococcus zooepidemicus, or from Corynebacterium pseudotuberculosis, Enterococcus faecalis, or Elizabethkingia meningoseptica, optionally wherein said enzyme is a EndoS, CP40, EndoE, or EndoF2 polypeptide.

5. The enzyme for use of claim 4, wherein:
- said IgG cysteine protease is a polypeptide having a sequence that is at least 80% identical to SEQ ID NO: 2, 4 or 5, such as at least 85%, 90%, 95% or 99% identical, or wherein said IgG cysteine protease comprises or consists of the sequence of any one of SEQ ID NOs: 6 to 25 and 55 to 69, optionally wherein said sequence includes an additional methionine at the N terminus and/or a histidine tag at the C terminus; or
- said IgG endoglycosidase is a polypeptide having a sequence that is at least 80% identical to SEQ ID NO: 90, such as at least 85%, 90%, 95% or 99% identical, optionally wherein said sequence includes an additional methionine at the N terminus and/or a histidine tag at the C terminus.

6. The enzyme for use of any one of the preceding claims wherein in the enzyme is imlifidase and/or EndoS.

7. The enzyme for use of any one of the preceding claims comprising one or more of:
(a) administration to the subject of a non-lethal dose of irradiation and/or any other agent which depletes the subject's HSPC;
(b) administration of an agent to reduce the numbers and/or down-modulate the activity of lymphocytes in the subject, wherein said lymphocytes include:
i. T cells; and/or
ii. B cells (optionally including antibody-producing cells);
(c) administration of any other agent or regimen which reduces the activity of the immune system, e.g., inhibitors of complement, inhibitors of cytokines, inhibitors of innate immune cells, inducers of tolerance.

8. The enzyme for use of claim 7 comprising at least (a) and (b), optionally wherein:
- (a) additionally comprises administration of an infusion of donor CD8-alpha cells; and/or
- (b) comprises administration of anti-CD117 and/or anti-CD47 antibodies; and/or
- (c) comprises the administration of ATG, anti-CD4, anti-CD8, anti-CD90 antibodies, anti-CD52 antibody, anti-CD117 antibody, anti-45 antibody, busulfan, cyclophosphamide, fludarabine, treosulfan, cyclosporin, tacrolimus, an immunotoxin targeting T-cells, anti-CD20 antibody, an anti-CD19 antibody, bortezomib, or an immunotoxin targeting B cells.

9. The enzyme for use of claim 7 or claim 8 comprising at least (a) and (b), wherein (b) comprises the administration of one or more of anti-CD4, anti-CD8 and anti-CD90 antibodies, bortezomib, cyclophosphamide, and/or rATG.

10. The enzyme for use of claim 7 or claim 8 comprising at least (a) and (b), wherein (b) comprises the administration of busulfan, and/or fludarabine.

11. The enzyme for use of any one of the preceding claims, further comprising a step of subsequently administering the HSPC to the subject in an amount sufficient and under conditions suitable to induce hematopoietic chimerism in the subject.

12. The enzyme for use according to claim 11, wherein the HSPC are allogeneic, syngeneic or autologous, and optionally are genetically modified.

13. An enzyme which inactivates serum IgG, for use in a method for the prevention or treatment of immune rejection of a cell, tissue or organ transplant wherein the method comprises a conditioning regimen for the transplant of hematopoietic stem and progenitor cells (HSPC) which comprises administering the enzyme to the subject and subsequently administering the HSPC to the subject in an amount sufficient and under conditions suitable to induce hematopoietic chimerism in the subject, optionally comprising administering a cell, tissue, or organ transplant to the subject from the same donor as the HSPC.

14. The enzyme for use of claim 13, wherein the transplant is kidney, liver, heart, pancreas, lung, small intestine, skin, blood vessels/vascular tissue, face, arm, trachea, parts of the eye, pancreatic islets, substantia nigra, bone marrow or stem cells, optionally including the HSPC used in the method for the induction of hematopoietic chimerism in a subject of claim 11 or 12 such that no additional transplant is required.

## Patentansprüche

1. Enzym, welches Serum-IgG-Moleküle in einem Patienten inaktiviert, zur Verwendung bei einem Konditionierungsregime für die Transplantation von hämatopoetischen Stamm- und Vorläuferzellen (HSPC) auf einen Patienten, umfassend das Verabreichen des Enyzms an den Patienten.

2. Enyzm zur Verwendung nach Anspruch 1, wobei die Menge des Enzyms ausreichend verabreicht wird, um alle oder im Wesentlichen alle IgG-Moleküle, die in dem Serum des Patienten vorhanden sind, zu inaktivieren.

3. Enzym zur Verwendung nach einem der vorherigen Ansprüche, wobei das Enzym eine IgG-Cysteinprotease oder eine IgG-Endoglycosidase ist.

4. Enzym zur Verwendung nach Anspruch 3, wobei:
(i) die IgG-Cysteinprotease von einem Streptococcus-Bakterium, wie Streptococcus pyogenes, stammt, wobei optional das Enzym ein IdeS-, IdeZ- oder MAC2-Polypeptid ist, oder
(ii) die IgG-Endoglycosidase von einem Streptococcus-Bakterium, wie Streptococcus pyogenes, Streptococcus equi oder Streptococcus zooepidemicus, oder von Corynebacterium pseudotuberculosis, Enterococcus faecalis oder Elizabethkingia meningoseptica stammt, wobei optional das Enzym ein EndoS-, CP40-, EndoE- oder EndoF2-Polypeptid ist.

5. Enzym zur Verwendung nach Anspruch 4, wobei:
- die IgG-Cysteinprotease ein Polypeptid ist, das eine Sequenz hat, die zu mindestens 80 % identisch mit SEQ ID NO: 2, 4 oder 5 ist, wie zu mindestens 85 %, 90 %, 95 % oder 99 % identisch, oder wobei die IgG-Cysteinprotease die Sequenz einer der SEQ ID NOs: 6 bis 25 und 55 bis 69 umfasst oder aus dieser besteht, wobei optional die Sequenz ein zusätzliches Methionin am N-Terminus und/oder ein Histidin-Tag am C-Terminus einschließt; oder
- die IgG-Endoglycosidase ein Polypeptid ist, das eine Sequenz hat, die zu mindestens 80 % identisch mit der SEQ ID NO: 90 ist, wie zu mindestens 85 %, 90 %, 95 % oder 99 % identisch, wobei optional die Sequenz ein zusätzliches Methionin am N-Terminus und/oder ein Histidin-Tag am C-Terminus einschließt.

6. Enzym zur Verwendung nach einem der vorherigen Ansprüche, wobei das Enzym Imlifidase und/oder EndoS ist.

7. Enzym zur Verwendung nach einem der vorherigen Ansprüche umfassend eine oder mehrere von:
(a) einer Verabreichung an den Patienten einer nicht-tödlichen Dosis von Bestrahlung und/oder eines beliebigen sonstigen Mittels, welches die HSPC des Patienten reduziert;
(b) einer Verabreichung eines Mittels zum Verringern der Anzahl und/oder Herabregulieren der Aktivität von Lymphozyten in dem Patienten, wobei die Lymphozyten Folgendes einschließen:
i. T-Zellen; und/oder
ii. B-Zellen (die optional antikörperproduzierende Zellen einschließen);
(c) einer Verabreichung eines sonstigen Mittels oder Regimes, welches die Aktivität des Immunsystems verringert, z. B. Komplementinhibitoren, Zytokininhibitoren, Inhibitoren angeborener Immunzellen, Toleranzinduktoren.

8. Enyzm zur Verwendung nach Anspruch 7, umfassend mindestens (a) und (b), wobei es optional:
- (a) zusätzlich die Verabreichung einer Infusion von Spender-CD8-Alpha-Zellen umfasst; und/oder
- (b) die Verabreichung von Anti-CD117- und/oder Anti-CD47-Antikörpern umfasst; und/oder
- (c) die Verabreichung von ATG-, Anti-CD4-, Anti-CD8-, Anti-CD90-Antikörpern, Anti-CD52-Antikörper, Anti-CD117-Antikörper, Anti-45-Antikörper, Busulfan, Cyclophosphamid, Fludarabin, Treosulfan, Cyclosporin, Tacrolimus, einem auf T-Zellen abzielenden Immunotoxin, einem Anti-CD20-Antikörper, einem Anti-CD19-Antikörper, Bortezomib, oder einem auf B-Zellen abzielenden Immunotoxin umfasst.

9. Enzym zur Verwendung nach Anspruch 7 oder 8 umfassend mindestens (a) und (b), wobei (b) die Verabreichung von einem oder mehreren von Anti-CD4-, Anti-CD8- und Anti-CD90-Antikörpern, Bortezomib, Cyclophosphamid und/oder rATG umfasst.

10. Enzym zur Verwendung nach Anspruch 7 oder Anspruch 8 umfassend mindestens (a) und (b), wobei (b) die Verabreichung von Busulfan und/oder Fludarabin umfasst.

11. Enzym zur Verwendung nach einem der vorherigen Ansprüche, ferner umfassend einen Schritt des sequenziellen Verabreichens der HSPC an den Patienten in einer ausreichenden Menge und unter Bedingungen, die zum Induzieren von hämatopoetischem Chimärismus bei dem Patienten geeignet sind.

12. Enzym zur Verwendung nach Anspruch 11, wobei die HSPC allogen, syngen oder autolog sind und optional genetisch modifiziert sind.

13. Enzym, welches Serum-IgG inaktiviert, zur Verwendung bei einem Verfahren zur Prävention oder Behandlung der Immunabstoßung einer Zelle, eines Gewebes oder Organtransplantats, wobei das Verfahren ein Konditionierungsregime für das Transplantat von hämatopoetischen Stamm- und Vorläuferzellen (HSPC) umfasst, welches das Verabreichen des Enzyms an den Patienten und das darauffolgende Verabreichen der HSPC an den Patienten in einer ausreichenden Menge und unter Bedingungen, die zum Induzieren von hämatopoetischem Chimärismus bei dem Patienten geeignet sind, umfasst, optional umfassend das Verabreichen eines Zell-, Gewebe- oder Organtransplantats an den Patienten von demselben Spender wie die HSPC.

14. Enzym zur Verwendung nach Anspruch 13, wobei das Transplantat eine Niere, eine Leber, ein Herz, eine Bauchspeicheldrüse, eine Lunge, ein Dünndarm, Haut, Blutgefäße/Gefäßgewebe, ein Gesicht, ein Arm, eine Luftröhre, Teile des Auges, Langerhans-Inseln, Substantia nigra, Knochenmark oder Stammzellen ist, das optional die HSPC einschließt, die bei dem Verfahren für die Induktion von hämatopoetischem Chimärismus bei einem Patienten nach Anspruch 11 der 12 verwendet werden, so dass kein zusätzliches Transplantat benötigt wird.

## Revendications

1. Enzyme qui inactive des molécules d'IgG sérique chez un sujet, destinée à être utilisée dans un régime de conditionnement pour le transplant de cellules souches et progénitrices hématopoïétiques (HSPC) à un sujet, comprenant l'administration de l'enzyme au sujet.

2. Enzyme destinée à être utilisée de la revendication 1, dans laquelle la quantité de ladite enzyme administrée est suffisante pour inactiver toutes ou sensiblement toutes les molécules d'IgG présentes dans le sérum du sujet.

3. Enzyme destinée à être utilisée d'une quelconque revendication précédente, dans laquelle l'enzyme est une protéase à cystéine IgG ou une endoglycosidase IgG.

4. Enzyme destinée à être utilisée de la revendication 3, dans laquelle :
(i) la protéase à cystéine IgG provient d'une bactérie streptocoque, telle que Streptococcus pyogenes, facultativement dans laquelle ladite enzyme est un polypeptide IdeS, IdeZ, ou MAC2, ou
(ii) l'endoglycosidase IgG provient d'une bactérie streptocoque, telle que Streptococcus pyogenes, Streptococcus equi, ou Streptococcus zooepidemicus, ou provient de Corynebacterium pseudotuberculosis, d'Enterococcus faecalis, ou d'Elizabethkingia meningoseptica, facultativement dans laquelle ladite enzyme est un polypeptide EndoS, CP40, EndoE, ou EndoF2.

5. Enzyme destinée à être utilisée de la revendication 4, dans laquelle :
- ladite protéase à cystéine IgG est un polypeptide ayant une séquence qui est au moins 80 % identique à SEQ ID n° : 2, 4 ou 5, par exemple au moins 85 %, 90 %, 95 % ou 99 % identique, ou dans laquelle ladite protéase à cystéine IgG comprend la séquence, ou en est constituée, d'un quelconque de SEQ ID n° : 6 à 25 et 55 à 69, facultativement dans laquelle ladite séquence inclut une méthionine supplémentaire à l'extrémité N-terminale et/ou une étiquette histidine à l'extrémité C-terminale ; ou
- ladite endoglycosidase IgG est un polypeptide ayant une séquence qui est au moins 80 % identique à SEQ ID n° : 90, par exemple au moins 85 %, 90 %, 95 % ou 99 % identique, facultativement dans laquelle ladite séquence inclut une méthionine supplémentaire à l'extrémité N-terminale et/ou une étiquette histidine à l'extrémité C-terminale.

6. Enzyme destinée à être utilisée de l'une quelconque des revendications précédentes, dans laquelle dans l'enzyme est imlifidase et/ou EndoS.

7. Enzyme destinée à être utilisée de l'une quelconque des revendications précédentes, comprenant une ou plusieurs parmi :
(a) l'administration au sujet d'une dose non léthale d'irradiation et/ou de tout autre agent qui entraîne la déplétion des HSPC du sujet ;
(b) l'administration d'un agent pour réduire les nombres et/ou effectuer la modulation descendance de l'activité de lymphocytes chez le sujet, dans laquelle lesdits lymphocytes incluent :
i. des cellules T ; et/ ou
ii. des cellules B (y compris facultativement des cellules productrices d'anticorps) ;
(c) l'administration de tout autre agent ou régime qui réduit l'activité du système immunitaires, par exemple des inhibiteurs de complément, des inhibiteurs de cytokines, des inhibiteurs de cellules immunitaires innées, des inducteurs de tolérance.

8. Enzyme destinée à être utilisée de la revendication 7, comprenant au moins (a) et (b), facultativement dans laquelle :
- (a) comprend de plus l'administration d'une infusion de cellules CD8-alpha de donneur ; et/ou
- (b) comprend l'administration d'anticorps anti-CD117 et/ou anti-CD47 ; et/ou
- (c) comprend l'administration de ATG, d'anticorps anti-CD4, anti-CD8, anti-CD90, d'un anticorps anti-CD52, d'un anticorps anti-CD117, d'un anticorps anti-45, de busulfan, de cyclophosphamide, de fludarabine, de tréosulfan, de cyclosporine, de tacrolimus, d'une immunotoxine ciblant des cellules T, d'un anticorps anti-CD20, d'un anti-CD19, de bortézomib, ou d'une immunotoxine ciblant des cellules B.

9. Enzyme destinée à être utilisée de la revendication 7 ou de la revendication 8 comprenant au moins (a) et (b), dans laquelle
(b) comprend l'administration d'un ou de plusieurs d'anticorps anti-CD4, anti-CD8, et anti-CD90, de bortézomib, de cyclophosphamide, et/ou de rATG.

10. Enzyme destinée à être utilisée de la revendication 7 ou de la revendication 8 comprenant au moins (a) et (b), dans laquelle
(b) comprend l'administration de busulfan, et/ou de fludarabine.

11. Enzyme destinée à être utilisée de l'une quelconque des revendications précédentes, comprenant en outre une étape, par la suite, de l'administration des HSPC au sujet en une quantité suffisante et dans des conditions appropriées pour induire un chimérisme hématopoïétique chez le sujet.

12. Enzyme destinée à être utilisée selon la revendication 11, dans laquelle les HSPC sont allogéniques, syngéniques, ou autologues, et facultativement sont génétiquement modifiées.

13. Enzyme qui inactive l'IgG sérique, destinée à être utilisée dans une méthode pour la prévention ou le traitement de rejet immunitaire d'un transplant cellulaire, tissulaire, ou d'organe, dans laquelle la méthode comprend un régime de conditionnement pour le transplant de cellules souches et progénitrices hématopoïétiques (HSPC) qui comprend l'administration de l'enzyme au sujet et par la suite l'administration des HSPC au sujet en une quantité suffisante et dans des conditions appropriées pour induire un chimérisme hématopoïétique chez le sujet, facultativement comprenant l'administration d'un transplant cellulaire, tissulaire, ou d'organe au sujet provenant du même donneur que les HSPC.

14. Enzyme destinée à être utilisée de la revendication 13, dans laquelle le transplant est le rein, le foie, le cœur, le pancréas, le poumon, l'intestin grêle, la peau, un vaisseau sanguin/tissu vasculaire, le visage, un bras, la trachée, des parties de l'œil, des îlots pancréatiques, la substance noire, la moëlle osseuse, ou des cellules souches, y compris facultativement les HSPC utilisées dans la méthode pour induire un chimérisme hématopoïétique chez un sujet de la revendication 11 ou 12 de manière telle qu'aucun transplant supplémentaire n'est requis.
